# EUROPEAN PATENT APPLICATION

(11) **EP 2 468 883 A1**
(43) Date of publication of application: **27.06.2012**
(21) Application number: 10382344.9
(22) Date of filing: 22.12.2010
(51) Int. Cl.: C12Q 1/68

(54) **Molecular biomarkers for predicting response to tyrosine kinase inhibitors in lung cancer**

(71) Applicant: Pangaea Biotech S.L., 08028 Barcelona (ES)
(72) Inventor: Tarón Roca, Miguel, E-08028 Barcelona (ES); Rosell Costa, Rafael, E-08028, Barcelona (ES)
(74) Representative: ABG Patentes, S.L.

(57) **Abstract**

The invention relates to methods for determining the clinical outcome of patients suffering lung cancer and being under treatment with an EGFR inhibitor. The methods are based on the detection of the presence of mutations in the EGFR gene conferring resistance to inhibitors of the EGFR tyrosine kinase activity, wherein the appearance of said mutations in the biofluid of the patient is indicative of a high probability that the patient suffers a relapse of the disease. The invention also provides therapeutic methods for said patients.

## Description

### FIELD OF THE INVENTION

The invention relates to the field of pharmacogenomics and, more in particular, to methods for predicting the clinical response of a lung cancer patient to an EGFR tyrosine kinase inhibitor-based chemotherapy, based on the detection of one sensitivity mutation in the EGFR gene towards an inhibitor of EGFR tyrosine kinase activity in a biofluid of the patient.

### BACKGROUND OF THE INVENTION

Non-small-cell lung cancer (NSCLC) accounts for approximately 80% of all lung cancers, with 1.2 million new cases worldwide each year. NSCLC resulted in more than one million deaths worldwide in 2001 and is the leading cause of cancer-related mortality in both men and women (31% and 25%, respectively). The prognosis of advanced NSCLC is dismal. A recent Eastern Cooperative Oncology Group trial of 1155 patients showed no differences among the chemotherapies used: cisplatin/paclitaxel, cisplatin/gemcitabine, cisplatin/docetaxel and carboplatin/paclitaxel. Overall median time to progression was 3.6 months, and median survival was 7.9 months.

At diagnosis, patients with NSCLC can be divided into three groups that reflect both the extent of the disease and the treatment approach:
■ The first group of patients has tumors that are surgically resectable (generally stage I, stage II, and selected stage III tumors). This group has the best prognosis.
■ The second group includes patients with either locally (T3-T4) and/or regionally (N2-N3) advanced lung cancer. Patients with unresectable or N2-N3 disease are treated with radiation therapy in combination with chemotherapy. Selected patients with T3 or N2 disease can be treated effectively with surgical resection and either preoperative or postoperative chemotherapy or chemoradiation therapy.
■ The final group includes patients with distant metastases (M1). This group can be treated with palliative radiation therapy or chemotherapy.

For the past several years, efforts have been focused on the development of targeted therapy direct against EGFR in non-small cell lung carcinoma (NSCLC). EGFR is present in the majority of NSCLCs. It is a member of the ErbB family of closely related receptors including EGFR (ErbB-1), Her2/neu (ErbB-2), Her3 (ErbB-3) and Her4 (ErbB-4). Activation of EGFR leads to receptor tyrosine kinase activation and a series of downstream signaling events that mediate increases in cellular proliferation, motility, adhesion, invasion, blocking of apoptosis and resistance to chemotherarapy. EGFR and its ligands, EGF and transforming growth factor alpha, are expressed in over 80% of NSCLC. Upon ligand binding, EGFR homodimerizes or forms heterodimers with other members of the ErbB family leading to receptor phosphorylation and activation of downstream signaling events. EGFR activation leads to the association with multiple signaling mediators such as She, Grb2, Src, JAKs, PLD, PLCGAMMA, and PI3K and subsequently to the activation of signaling transducers such as ERK1/2, FAK, JNK, STATs, and Akt. The importance of EGFR in tumorigenesis has prompted the development and commercialization of therapeutic agents that block its function.

The recent treatment success of gefitinib (Iressa) and erlotinib (Tarceva), two small molecule inhibitors of EGFR, in a fraction of patients with NSCLC has further solidified the premise that EGFR is a valid target. Several groups have independently identified frequent somatic mutations in the kinase domain of the EGFR gene in lung adenocarcinoma. These occur in 16% of lung adenocarcinoma specimens sequenced in the U.S. and 40% of those sequenced in Asia. The mutations are associated with sensitivity to both gefitinib and erlotinib, explaining in part the rare and dramatic clinical responses to treatment with these agents. Subsequent studies by multiple groups have now identified EGFR kinase domain mutations from many additional lung cancer patients. These mutations cluster in four groups, or regions; exon 19 deletions, exon 20 insertions, and point mutations at G719S and L858R. Thus far, the incidence of these kinase domain mutations is more common in adenocarcinomas than in lung cancers of other histological subtypes such as squamous cell carcinoma. Recent emerging data also suggest that EGFR expression assessed by immunohistochemistry and the EGFR gene copy number might play an equally important role in identifying patients more likely to respond and have longer survival when treated with gefitinib or erlotinib.

However, nearly all patients who initially respond to erlotinib and gefitinib subsequently relapse. The importance of indentifying early in time the patients that are suffering or will suffer a relapse before the physical relapse symptoms appear (cough, pain or tumoral mass observed by PET/CT) is very high since it will allow the practitioners to choose an alternative therapy. An early diagnosis of the relapse will result in an increase of the survival rate of lung cancer patients.

Thus, there is a need in the art for further prognosis tools for predicting the relapse in a patient suffering with lung cancer before the physical relapse symptoms appear.

### SUMMARY OF THE INVENTION

In a first aspect, the invention relates to a method for predicting the clinical response of a patient suffering lung cancer to an EGFR inhibitor-based therapy comprising
(i) determining at a first time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid sequence of the non mutated EGFR gene and
(ii) determining at a second time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid of the non mutated EGFR gene,
   wherein said second time point is later than the first time point and
   wherein an increase in the ratio determined at the second time point with respect to the ratio determined at the first time point is indicative of a negative clinical response of said patient to said EGFR inhibitor-based therapy or
   wherein a decrease in the ratio determined at the second time point with respect to the ratio at determined at the first time point is indicative of a positive clinical response of said patient to said EGFR inhibitor-based therapy.

In a second aspect, the invention relates to a composition comprising an EGFR inhibitor for use in the treatment of lung cancer in a patient suffering lung cancer wherein said patient is selected by a method comprising
(i) determining at a first time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid sequence of the non mutated EGFR gene and
(ii) determining at a second time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid of the non mutated EGFR gene,
wherein said second time point is later than the first time point and
wherein the ratio determined at the second time point is increased with respect to the ratio at determined at the first time point.

### BRIEF DESCRIPTION OF THE FIGURES

**Figure 1**: Data for patient DX282 corresponding to a 40 years old female, light smoker suffering from stage IV lung adenocarcinoma (live, bone).
**Figure 2**: Data for Patient DX271 corresponding to a 38 years old male, non-smoker suffering from lung stage IV adenocarcinoma (lung, lymph nodes).
**Figure 3**: Data for patient DX104 corresponding to a 42 years old male, non-smoker. suffering from large cell carcinoma stage IV (brain, bone, liver, lymph nodes).
**Figure 4**: Data from patient DX138, corresponding to a 44 years old female, non-smoker suffering from stage IV lung adenocarcinoma (2 Brain mt).

### DETAILED DESCRIPTION OF THE INVENTION

### Method for determining the clinical outcome of a patient

The authors of the present invention have observed that the relapse of patients which suffer lung cancer and which are treated with an inhibitor of EGFR tyrosine kinase can be determined even before the appearance of clinical symptoms appear or before the confirmation of progression/relapse with imaging data by detecting an increase in the appearance in a biofluid of the patient of copies of the nucleic acid encoding EGFR carrying one or more mutations conferring conferring sensitivity to EGFR tyrosine kinase inhibitors. As shown in the examples of the present invention, the appearance of mutations in the EGFR gene associated with sensitivity to EGFR TK inhibitors in a biofluid of the patient precedes the relapse of the disease as observed by the appearance of clinical symptoms and, conversely, the absence of these types of mutations correlates with the absence of relapse and the lack of evidence of disease.

Thus, this method is particularly suited as a high-sensitive method for monitoring of patients after surgical or chemical response for their risk of suffering relapse.

Thus, in a first aspect, the invention relates to a method (hereinafter first method of the invention) for predicting the clinical response of a patient suffering lung cancer to an EGFR inhibitor-based therapy comprising
(i) determining at a first time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid sequence of the non mutated EGFR gene and
(ii) determining at a second time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid of the non mutated EGFR gene,
wherein said second time point is later than the first time point and
wherein an increase in the ratio determined at the second time point with respect to the ratio determined at the first time point is indicative of a negative clinical response of said patient to said EGFR inhibitor-based therapy or
wherein a decrease in the ratio determined at the second time point with respect to the ratio at determined at the first time point is indicative of a positive clinical response of said patient to said EGFR inhibitor-based therapy

The term "predicting", as used herein, as used herein, refers to the determination of the likelihood that the patient will respond either favorably or unfavorably to a given therapy. Especially, the term "prediction", as used herein, relates to an individual assessment of any parameter that can be useful in determining the evolution of a patient. As will be understood by those skilled in the art, the prediction of the clinical response to the treatment with a biological drug , although preferred to be, need not be correct for 100% of the subjects to be diagnosed or evaluated. The term, however, requires that a statistically significant portion of subjects can be identified as having an increased probability of having a positive response. Whether a subject is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90% at least 95%. The p-values are, preferably, 0.2, 0.1 or 0.05.

The term "clinical response", as used herein, refers to the response to a biological drug of the subject suffering from a pathology which is treatable with said biological. Standard criteria may vary from disease to disease. It denotes the doctor's prediction of how a subject's disease will progress, and whether there is chance of recovery or recurrence.

The term "patient", as used herein, refers to all animals classified as mammals and includes, but is not restricted to, domestic and farm animals, primates and humans, e.g., human beings, non-human primates, cows, horses, pigs, sheep, goats, dogs, cats, or rodents. Preferably, the patient is a male or female human of any age or race. In an embodiment the patient that has had lung cancer still is considered to have lung cancer.

In a preferred embodiment, the patient which is screened according to the first method of the invention is characterized in that a tissue sample from the tumor of said patient at the first time point is positive for a sensitivity mutation of the EGFR gene towards an inhibitor of tyrosine kinase activity. The term "sensitivity mutation of the EGFR gene towards an inhibitor of tyrosine kinase activity" is defined in detail below.

In a preferred embodiment, the patient shows no relapse symptoms at either the first and/or second time points. In a still more preferred embodiment, the patient shows relapse symptoms neither at the first nor at the second time points. Relapse symptoms that can be used as a criteria for positive response are cough, pain or tumoral mass observed by PET/CT.

The term "lung cancer" is meant to refer to any cancer of the lung and includes non-small cell lung carcinomas and small cell lung carcinomas. In a preferred embodiment, the methods of the invention are applicable to a subject suffering from NSCLC and/or that has suffered NSCLC. In a particular embodiment, the NSCLC is selected from squamous cell carcinoma of the lung, large cell carcinoma of the lung, and adenocarcinoma of the lung. Furthermore, the present method can also be applicable to a subject that has suffered or is suffering from any stage of NSCLC (stages 0, IA, IB, IIa, IIb, IIIa, IIIb o IV). In a preferred embodiment, the patient has had advanced lung cancer.

The term "EGFR inhibitor-based chemotherapy", as used herein, refers to any therapeutic regime which includes one or more compounds capable of inhibiting the activity of EGFR.

The terms "EGFR", "ErbB1" and "epiderma1 growth factor receptor" and are used interchangeably herein and refer to a tyrosine kinase which regulate signaling pathways and growth and survival of cells and which shows affinity for the EGF molecule. The ErbB family of receptors consists of four closely related subtypes: ErbB1 (epidermal growth factor receptor [EGFR]), ErbB2 (HER2/neu), ErbB3 (HER3), and ErbB4 (HER4) and variants thereof (e.g. a deletion mutant EGFR as in Humphrey et al. (Proc. Natl. Acad. Sci. USA, 1990, 87:4207-4211). In a preferred embodiment, the EGFR is human.

In a preferred embodiment, the therapeutic regime is an EGFR tyrosine kinase inhibitor. The type of EGFR tyrosine kinase inhibitor therapy for use according to the method of the present invention is not particularly limiting and may include any of the inhibitors mentioned above. In a preferred embodiment, the EGFR tyrosine kinase inhibitor is a dual EGFR inhibitor, a dual EGFR tyrosine kinase inhibitor or a EGFR tyrosine kinase inhibitor specific for EGFR carrying a resistance mutation.

The term "dual EGFR inhibitor", as used herein, refers to a composition which is capable of simultaneously inhibiting the tyrosine kinase activity of the intracellular domain of EGFR as well as its activation by the binding of the ligand to the extracellular domain. Illustrative and non-limitaative example of such an inhibitor is, e.g. the composition comprising cetuximab (C225) as inhibitor of the extracellular domain and erlotinib (E) as inhibitor of the tyrosine kinase activity of the intracellular domain.

The term "dual EGFR tyrosine kinase inhibitor", as used herein, refers to a compound which is capable of simultaneously inhibiting EGFR and HER2 activity. Examples of such compounds include the EGFR and HER2 inhibitor CI-1033 (formerly known as PD183805; Pfizer); the EGFR and HER2 inhibitor GW-2016 (also known as GW-572016 or lapatinib ditosylate; GSK); the EGFR and JAK 2/3 inhibitor AG490 (a tyrphostin); the EGFR and HER2 inhibitor ARRY-334543 (Array BioPharma); BIBW-2992, an irreversible dual EGFR/HER2 kinase inhibitor (Boehringer Ingelheim Corp.)

In a preferred embodiment, the EGFR inhibitor-based chemotherapy is an inhibitor of the EGFR tyrosine kinase. The expression "EGFR tyrosine kinase inhibitor", as used herein, relates to a chemical substance inhibiting "tyrosine kinase" which transfers a γ-phosphate group of ATP to a hydroxy group of a specific tyrosine in protein catalised by the tyrosine kinase domain of the receptor for epidermal growth factor (EGFR). Tyrosine kinase activity is measured by detecting phosphorylation of a protein. EGFR tyrosine kinase inhibitors are known in the art. For example, a tyrosine kinase inhibitor is identified by detecting a decrease the tyrosine mediated transfer phosphate from ATP to protein tyrosine residues.

The tyrosine kinase inhibitor is for example an erbB tyrosine kinase inhibitor. Alternatively the tyrosine kinase inhibitor is an EGFR tyrosine kinase inhibitor. The tyrosine kinase inhibitor is a reversible tyrosine kinase inhibitor. Alternatively the tyrosine kinase inhibitor is an irreversible tyrosine kinase inhibitor. Reversible tyrosine kinase inhibitors include for example, HKI-272, BIBW2992, EKB-569 or CL-387,785 or mimetics or derivatives thereof. Other tyrosine kinase inhibitors include those described in U.S. Pat. Nos. 6,384,051, 6,288,082 and US Application No. 20050059678, each of which is hereby incorporated by reference in their entireties.

EGFR tyrosine kinase inhibitors include, for example quinazoline EGFR kinase inhibitors, pyrido- pyrimidine EGFR kinase inhibitors, pyrimido-pyrimidine EGFR kinase inhibitors, pyrrolo- pyrimidine EGFR kinase inhibitors, pyrazolo-pyrimidine EGFR kinase inhibitors, phenylamino- pyrimidine EGFR kinase inhibitors, oxindole EGFR kinase inhibitors, indolocarbazole EGFR kinase inhibitors, phthalazine EGFR kinase inhibitors, isoflavone EGFR kinase inhibitors, quinalone EGFR kinase inhibitors, and tyrphostin EGFR kinase inhibitors, such as those described in the following patent publications, and all pharmaceutically acceptable salts and solvates of said EGFR kinase inhibitors: International Patent Publication Nos. WO 96/33980, WO 96/30347, WO 97/30034, WO 97/30044, WO 97/38994, WO 97/49688, WO 98/02434, WO 97/38983, WO 95/19774, WO 95/19970, WO 97/13771, WO 98/02437, WO 98/02438, WO 97/32881, WO 98/33798, WO 97/32880, WO 97/3288, WO 97/02266, WO 97/27199, WO 98/07726, WO 97/34895, WO 96/31510, WO 98/14449, WO 98/14450, WO 98/14451, WO 95/09847, WO 97/19065, WO 98/17662, WO 99/35146, WO 99/35132, WO 99/07701, and WO 92/20642; European Patent Application Nos. EP 520722, EP 566226, EP 787772, EP 837063, and EP 682027; U.S. Pat. Nos. 5,747,498, 5,789,427, 5,650,415, and 5,656,643; and German Patent Application No. DE 19629652. Additional nonlimiting examples of low molecular weight EGFR kinase inhibitors include any of the EGFR tyrosine kinase inhibitors described in Traxler, P., 1998, Exp. Opin. Ther. Patents 8(12): 1599-1625.

Specific preferred examples of low molecular weight EGFR tyrosine kinase inhibitors that can be used according to the present invention include [6,7-bis(2-methoxyethoxy)-4-quinazolin-4-yl]- (3-ethynylphenyl)amine (also known as OSI-774, erlotinib, or TARCEVA.RTM. (erlotinib HCl); OSI Pharmaceuticals/Genentech/Roche) (U.S. Pat. No. 5,747,498; International Patent Publication No. WO 01/34574, and Moyer, J. D. et al. (1997) Cancer Res. 57:4838-4848); CI- 1033 (formerly known as PD183805; Pfizer) (Sherwood et al., 1999, Proc. Am. Assoc. Cancer Res. 40:723); PD-158780 (Pfizer); AG-1478 (University of California); CGP-59326 (Novartis); PKI-166 (Novartis); EKB-569 (Wyeth); GW-2016 (also known as GW-572016 or lapatinib ditosylate; GSK); and gefitinib (also known as ZD1839 or IRESSA.TM.; Astrazeneca) (Woodburn et al., 1997, Proc. Am. Assoc. Cancer Res. 38:633). A particularly preferred low molecular weight EGFR kinase inhibitor that can be used according to the present invention is [6,7-bis(2-methoxyethoxy)-4-quinazolin-4-yl]-(3-ethynylphenyl) amine (i.e. erlotinib), its hydrochloride salt (i.e. erlotinib HCl, TARCEVA.RTM.), or other salt forms (e.g. erlotinib mesylate).

EGFR tyrosine kinase inhibitors also include, for example multi-kinase inhibitors that have activity on EGFR kinase, i.e. inhibitors that inhibit EGFR kinase and one or more additional kinases. Examples of such compounds include the EGFR and HER2 inhibitor CI- 1033 (formerly known as PD 183805; Pfizer); the EGFR and HER2 inhibitor GW-2016 (also known as GW- 572016 or lapatinib ditosylate; GSK); the EGFR and JAK 2/3 inhibitor AG490 (a tyrphostin); the EGFR and HER2 inhibitor ARRY-334543 (Array BioPharma); BIBW-2992, an irreversible dual EGFR/HER2 kinase inhibitor (Boehringer Ingelheim Corp.); the EGFR and HER2 inhibitor EKB-569 (Wyeth); the VEGF-R2 and EGFR inhibitor ZD6474 (also known as ZACTIMA.TM.;AstraZeneca Pharmaceuticals), and the EGFR and HER2 inhibitor BMS-599626 (Bristol-Myers Squibb).

Antibody-based tyrosine EGFR kinase inhibitors include any anti-EGFR antibody or antibody fragment that can partially or completely block EGFR activation by its natural ligand. Non- limiting examples of antibody-based EGFR kinase inhibitors include those described in Modjtahedi, H., et al., 1993, Br. J. Cancer 67:247-253; Teramoto, T., et al., 1996, Cancer 77:639-645; Goldstein et al., 1995, Clin. Cancer Res. 1:1311-1318; Huang, S. M., et al., 1999, Cancer Res. 15:59(8):1935-40; and Yang, X., et al., 1999, Cancer Res. 59:1236-1243. Thus, the EGFR kinase inhibitor can be the monoclonal antibody Mab E7.6.3 (Yang, X. D. et al. (1999) Cancer Res. 59: 1236-43), or Mab C225 (ATCC Accession No. HB-8508), or an antibody or antibody fragment having the binding specificity thereof. Suitable monoclonal antibody EGFR kinase inhibitors include, but are not limited to, IMC-C225 (also known as cetuximab or ERBITUX.TM.; Imclone Systems), ABX-EGF (Abgenix), EMD 72000 (Merck KgaA, Darmstadt), RH3 (York Medical Bioscience Inc.), and MDX-447 (Medarex/Merck KgaA).

In another embodiment, an antisense strategy may be used to interfere with the kinase activity of a variant EGFR. This approach may, for instance, utilize antisense nucleic acids or ribozymes that block translation of a specific mRNA, either by masking that mRNA with an antisense nucleic acid or cleaving it with a ribozyme. For a general discussion of antisense technology, see, e.g., Antisense DNA and RNA, (Cold Spring Harbor Laboratory, D. Melton, ed., 1988).

Reversible short inhibition of variant EGFR gene transcription may also be useful. Such inhibition can be achieved by use of siRNAs. RNA interference (RNAi) technology prevents the expression of gene by using small RNA molecules such as small interfering RNAs (siRNAs). This technology in turn takes advantage of the fact that RNAi is a natural biological mechanism for silencing genes in most cells of many living organisms, from plants to insects to mammals (McManus et al., Nature Reviews Genetics, 2002, 3(10) p. 737). RNAi prevents a gene from producing a functional protein by ensuring that the molecule intermediate, the messenger RNA copy of the gene is destroyed. siRNAs can be used in a naked form and incorporated in a vector, as described below. One can further make use of aptamers to specifically inhibit variant EGFR gene transcription, see, for example, U.S. Patent 6,699,843. Aptamers useful in the present invention may be identified using the SELEX process. The methods of SELEX have been described in, for example, U. S. Patent Nos. 5,707,796, 5,763,177, 6,011,577, 5,580,737, 5,567,588, and 5,660,985.

An "antisense nucleic acid" or "antisense oligonucleotide" is a single stranded nucleic acid molecule, which, on hybridizing under cytoplasmic conditions with complementary bases in a RNA or DNA molecule, inhibits the latter's role. If the RNA is a messenger RNA transcript, the antisense nucleic acid is a counter-transcript or mRNA-interfering complementary nucleic acid. As presently used, "antisense" broadly includes RNA-RNA interactions, RNA- DNA interactions, ribozymes, RNAi, aptamers and Rnase-H mediated arrest.

Ribozymes are RNA molecules possessing the ability to specifically cleave other single stranded RNA molecules in a manner somewhat analogous to DNA restriction endonucleases. Ribozymes were discovered from the observation that certain mRNAs have the ability to excise their own introns. By modifying the nucleotide sequence of these ribozymes, researchers have been able to engineer molecules that recognize specific nucleotide sequences in an RNA molecule and cleave it (Cech, 1989, Science 245(4915) p. 276). Because they are sequence-specific, only mRNAs with particular sequences are inactivated.

Antisense nucleic acid molecules can be encoded by a recombinant gene for expression in a cell (e.g., U.S. patent No 5,814,500; U.S. 5,811,234), or alternatively they can be prepared synthetically (e.g., u.s. patent No 5,780,607).

siRNAs have been described in Brummelkamp et al., Science 296; 550-553,2002, Jaque et al., Nature 418; 435-438, 2002, Elbashir S. M. et al. (2001) Nature, 411: 494-498, McCaffrey et al. (2002), Nature, 418: 38-39; Xia H. et al. (2002), Nat. Biotech. 20: 1006-1010, Novina et al. (2002), Nat. Med. 8: 681-686, and U.S. Application No. 20030198627.

An important advantage of such a therapeutic strategy relative to the use of drugs such as gefitinib or erlotinib, which inhibit both the mutated receptor and the normal receptor, is that siRNA directed specifically against the mutated EGFR should not inhibit the wild-type EGFR. This is significant because it is generally believed that the "side effects" of gefitinib treatment, which include diarrhea and dermatitis, are a consequence of inhibition of EGFR in normal tissues that require its function.

In another embodiment, the compounds are antisense molecules specific for human sequences coding for an EGFR having at least one variance in its kinase domain. The administered therapeutic agent may be an antisense oligonucleotides, particularly synthetic oligonucleotides; having chemical modifications from native nucleic acids, or nucleic acid constructs that express such anti-sense molecules as RNA. The antisense sequence is complementary to the mRNA of the targeted EGFR genes, and inhibits expression of the targeted gene products (see e.g. Nyce et al. (1997) Nature 385:720). Antisense molecules inhibit gene expression by reducing the am.ount ofmRNA available for translation, through activation of RNAse H or steric hindrance. One or a combination of antisense molecules may be administered, where a combination may comprise multiple different sequences from a single targeted gene, or sequences that complement several different genes.

A preferred target gene is an EGFR with at least one nucleic acid variance in its kinase domain. Generally, the antisense sequence will have the same species of origin as the animal host.

Antisense molecules may be produced by expression of all or a part of the target gene sequence in an appropriate vector, where the vector is introduced and expressed in the targeted cells. The transcriptional initiation will be oriented such that the antisense strand is produced as an RNA molecule. The anti-sense RNA hybridizes with the endogenous sense strand mRNA, thereby blocking expression of the targeted gene. The native transcriptional initiation region, or an exogenous transcriptional initiation region may be employed.

The promoter may be introduced by recombinant methods in vitro, or as the result of homologous integration of the sequence into a chromosome. Many strong promoters that are active in muscle cells are known in the art, including the β-actin promoter, SV40 early and late promoters, human cytomegalovirus promoter, retroviral LTRs, etc. Transcription vectors generally have convenient restriction sites located near the promoter sequence to provide for the insertion of nucleic acid sequences. Transcription cassettes maybe prepared comprising a transcription initiation region, the target gene or fragment thereof, and a transcriptional termination region. The transcription cassettes may be introduced into a variety of vectors, e.g. plasmid; retrovirus, e.g. lentivirus; adenovirus; and the like, where the vectors are able to transiently or stably be maintained in cells, usually for a period of at least about one day, more usually for a period of at least about several days.

Aptamers are also useful. Aptamers are a promising new class of therapeutic oligonucleotides or peptides and are selected in vitro to specifically bind to a given target with high affinity, such as for example ligand receptors. Their binding characteristics are likely a reflection of the ability of oligonucleotides to form three dimensional structures held together by intramolecular nucleobase pairing. Aptamers are synthetic DNA, RNA or peptide sequences which may be normal and modified (e.g. peptide nucleic acid (PNA), thiophophorylated DNA, etc) that interact with a target protein, ligand (lIpid, carbohydrate, metabolite, etc). In a further embodiment, RNA aptamers specific for a variant EGFR can be introduced into or expressed in a cell as a therapeutic.

Peptide nucleic acids (PNAs) are compounds that in certain respects are similar to oligonucleotides and their analogs and thus may mimic DNA and RNA. In PNA, the deoxyribose backbone of oligonucleotides has been replaced by a pseudo-peptide backbone (Nielsen et al. 1991 Science 254, 1457-1500). Each subunit, or monomer, has a naturally occurring or non-naturally occurring nucleobase attached to this backbone. One such backbone is constructed of repeating units of N(2-aminoethyl) glycine linked through amide bonds. PNA hybridises with complementary nucleic acids through Watson and Crick base pairing and helix fold. The Pseudo-peptide backbone provides superior hybridization properties (Egholm et al. Nature (1993) 365, 566-568), resistance to enzymatic degradation (Demidov et al. Biochem. Pharmacol. (1994) 48, 1310-1313) and access to a variety of chemical modifications (Nielsen and Haaima Chemical Society Reviews (1997) 73-78). PNAs specific for a variant EGFR can be introduced into or expressed in a cell as a therapeutic. PNAs have been described, for example, in U.S. Application No. 20040063906.

In a preferred embodiment, the EGFR tyrosine kinase inhibitor is erlotinib or gefitinib.

The first method of the invention is suitable for predicting the response of lung cancer patient carrying at least one sensitivity mutation of EGFR towards an inhibitor of EGFR tyrosine kinase activity both when the tyrosine kinase inhibitor is used as first line treatment in patients which have not been previously treated with chemotherapy as well as when the EGFR tyrosine kinase inhibitor is used as second line in patients which have been previously been treated with conventional chemotherapy but which did not respond or ceased to respond.

The term "first-line treatment" or "first-line therapy" as used herein is an art recognized term and is understood to refer to the first chemotherapy treatment of cancer, which may be combined with surgery and/or radiation therapy, also called primary treatment or primary therapy. Typical antitumor compounds that can be used as first line for the treatment of lung cancer include, but are not limited to, plant alkaloids, such as vincristine, vinblastine and etoposide; anthracycline antibiotics including doxorubicin, epirubicin, daunorubicin; fluorouracil; antibiotics including bleomycin, mitomycin, plicamycin, dactinomycin; topoisomerase inhibitors, such as camptothecin and its analogues; and platinum compounds, including cisplatin and its analogues, such as carboplatin. Other traditional chemotherapeutic agents suitable for use are known to those of skill in the art and include, asparaginase, busuffan, chlorambucil, cyclophosphamide, cytarabine, dacarbazine, estramustine phosphate sodium, floxuridine, fluorouracil (5-FU), hydroxyurea (hydroxycarbamide), ifosfamide, lornustine (CCNU), mechlorethamine HCl (nitrogen mustard), melphalan, mercaptopurine, methotrexate (MTX), mitomycin, mitotane, mitsxantrone" procarbazine, streptozocin" thioguanine, thiotepa, amsacrine (m-AMSA), azacitidine" hexamethylmeiamine (HMM)" mitoguazone (methyl-GAG; methyl giyoxal bis- guanyihydrazone; MGBG), semustine (methyi-CCNU), teniposide (VM-26) and vindesine sulfate.

The term "second-line treatment" or "second-line therapy" as used herein is an art recognized term and is understood to refer to a chemotherapy treatment that is given when initial or primary treatment (first-line or primary therapy) doesn't work, or stops working.

In a preferred embodiment, the patients who are screened according to the first method of the invention have been treated by surgery prior to the determination at the first time point. Typically, surgery is used to remove the tumor along with some surrounding lung tissue and may include a segmentectomy or wedge resection wherein only part of a bobe is removed, a lobectomy, wherein a lobe (section) of the lung being removed or a pneumonectomy wherein the entire lung is removed.

In a first step, the method for predicting the clinical outcome of a patient comprises the determination in a bio-fluid of said patient of the ratio between the number of copies of the nucleic acid sequence of the EGFR which contains at least one sensitivity mutation towards an inhibitor of EGFR tyrosine kinase activity and the number of copies of the nucleic acid sequence of the non mutated EGFR gene at a first time point.

The term "bio-fluid" as used herein, relates to any fluid sample which can be obtained from the subject. Samples may be collected from a variety of sources from a mammal (e.g., a human), including a body fluid sample, blood, serum, sputum including saliva, plasma, nipple aspirants, synovial fluids, cerebrospinal fluids, sweat, urine, fecal matter, pancreatic fluid, trabecular fluid, cerebrospinal fluid, tears, bronchial lavage, swabbings, bronchial aspirants, semen, prostatic fluid, precervicular fluid, vaginal fluids, pre-ejaculate, etc. In a preferred embodiment, the bio-fluid is blood or serum.

In the practice of the invention bio-fluid such as blood is drawn by standard methods into a collection tube. In the case of blood, said tube preferably comprises siliconized glass, either without anticoagulant for preparation of serum or with EDTA, heparin, or similar anticoagulants, most preferably EDTA, for preparation of plasma. Plasma may optionally be subsequently converted to serum by incubation of the anticoagulated plasma with an equal volume of calcium chloride at 37°C for a brief period, most preferably for 1-3 minutes, until clotting takes place. The clot may then be pelleted by a brief centrifugation and the deproteinized plasma removed to another tube. Alternatively, the centrifugation may be omitted. Serum can also be obtained using clot activator tubes.

The term "nucleic acid" refers to a multimeric compound comprising nucleosides or nucleoside analogues which have nitrogenous heterocyclic bases, or base analogues, which are linked by phosphodiester bonds to form a polynucleotide such as DNA.

The term "DNA" refers to deoxyribonucleic acid. A DNA sequence is a deoxyribonucleic sequence. DNA is a long polymer of nucleotides and encodes the sequence of the amino acid residues in proteins using the genetic code.

The term "mutation in the EGFR gene conferring sensitivity of EGFR towards an inhibitor of EGFR tyrosine kinase activity" or "mutations conferring sensitivity to EGFR tyrosine kinase inhibitors", as used herein, refer to mutants in the tyrosine kinase domain of EGFR which result in an increased inhibition of the tyrosine kinase activity of EGFR in response to the treatment with inhibitor such as erlotinib. EGFR mutants showing an increased sensitivity to tyrosine kinase inhibitors include, without limitation, mutations at positions L858 in exon 21 such as L858R, L858P, L861Q or L861 point mutations in the activation loop (exon 21), in-frame deletion/insertion mutations in the ELREA sequence (exon 19) such as the E746-R748 deletion, the E746-A750 deletion, the E746-R748 deletion together with E749Q and A750P substitutions, del L747-E749 deletion combined with the A750P substitution, the L747S substitution in combination with the R748-P753 deletion, the L747-S752 deletion in combination with the E746V substitution, the L747-T751 deletion combined with an serine insertion, the AI insertion at positions M766-A767, the SVA insertion at positions S768-V769, or substitutions in at position 719 in the nucleotide binding loop (exon 18) such as G719A, G719C, G710S.

In a preferred embodiment, the patient shows at least a mutation conferring sensitivity to tyrosine kinase inhibitors. In a still more preferred embodiment, the patient shows a first mutation selected from the group of the L858R substitution and the ELREA deletion.

The detection of mutant nucleic acids encoding EGFR can be based on detection of mutations in genomic DNA, as well as transcripts and proteins themselves. It can be desirable to confirm mutations in genomic DNA by analysis of transcripts and/or polypeptides, in order to ensure that the detected mutation is indeed expressed in the subject.

Mutations in genomic nucleic acid are advantageously detected by techniques based on mobility shift in amplified nucleic acid fragments. For instance, Chen et al. (Anal [Biochem., 1996, 239:61-9), describe the detection of single-base mutations by a competitive mobility shift assay. Moreover, assays based on the technique of Marcelino et ai., BioTechniques 26(6): 1134-1148 (June 1999) are available commercially. In a preferred example, capillary heteroduplex analysis may be used to detect the presence of mutations based on mobility shift of duplex nucleic acids in capillary systems as a result of the presence of mismatches.

Generation of nucleic acids for analysis from samples generally requires nucleic acid amplification. Many amplification methods rely on an enzymatic chain reaction (such as a polymerase chain reaction, a ligase chain reaction, or a self-sustained sequence replication) or from the replication of all or part of the vector into which it has been cloned. Preferably, the amplification according to the invention is an exponential amplification, as exhibited by for example the polymerase chain reaction.

Many target and signal amplification methods have been described in the literature, for example, general reviews of these methods in Landegren, U., et al., Science 242:229- 237 (1988) and Lewis, R., Genetic Engineering News 10:1, 54-55 (1990). These amplification methods can be used in the methods of our invention, and include polymerase chain reaction (PCR), PCR in situ, ligase amplification reaction (LAR), ligase hybridisation, Qbeta bacteriophage replicase, transcription-based amplification system (TAS), genomic amplification with transcript sequencing (GAWTS), nucleic acid sequence-based amplification (NASBA) and in situ hybridisation. Primers suitable for use in various amplification techniques can be prepared according to methods known in the art.

Once the nucleic acid has been amplified, a number of techniques are available for detection of single base pair mutations. One such technique is Single Stranded Conformational Polymorphism (SSCP). SCCP detection is based on the aberrant migration of single stranded mutated DNA compared to reference DNA during electrophoresis. Mutation produces conformational change in single stranded DNA, resulting in mobility shift. Fluorescent SCCP uses fluorescent-labelled primers to aid detection. Reference and mutant DNA are thus amplified using fluorescent labelled primers. The amplified DNA is denatured and snap-cooled to produce single stranded DNA molecules, which are examined by non-denaturing gel electrophoresis.

Chemical mismatch cleavage (CMC) is based on the recognition and cleavage of DNA mismatched base pairs by a combination of hydroxylamine, osmium tetroxide and piperidine. Thus, both reference DNA and mutant DNA are amplified with fluorescent labelled primers. The amplicons are hybridised and then subjected to cleavage using Osmium tetroxide, which binds to an mismatched T base, or Hydroxylamine, which binds to mismatched C base, followed by Piperidine which cleaves at the site of a modified base. Cleaved fragments are then detected by electrophoresis.

Techniques based on restriction fragment polymorphisms (RFLPs) can also be used. Although many single nucleotide polymorphisms (SNPs) do not permit conventional RFLP analysis, primer-induced restriction analysis PCR (PIRA-PCR) can be used to introduce restriction sites using PCR primers in a SNP-dependent manner. Primers for PIRA-PCR which introduce suitable restriction sites can be designed by computational analysis, for example as described in Xiaiyi eif a/., (2001) Bioinformatics 17:838-839.

Furthermore, techniques based on WAVE analysis can be used (Methods Mol. Med. 2004; 108: 173-88). This system of DNA fragment analysis can be used to detect single nucleotide polymorphisms and is based on temperature-modulated liquid chromatography and a high-resolution matrix (Genet Test. 1997-98;1 (3):201-6.)

Real-time PCR (also known as Quantitative PCR, Real-time Quantitative PCR, or RTQ- PCR) is a method of simultaneous DNA quantification and amplification (Expert Rev. MoI. Diagn. 2005(2):209-19). DNA is specifically amplified by polymerase chain reaction. After each round of amplification, the DNA is quantified. Common methods of quantification include the use of fluorescent dyes that intercalate with double-strand DNA and modified DNA oligonucleotides (called probes) that fluoresce when hybridised with a complementary DNA.

In a particular embodiment of the invention, the detecting step of the method of the invention is carried out by means of nucleic acid sequencing. Illustrative, non limitative, examples of nucleic acid sequencing methods are cycle sequencing (Sarkar et al., 1995, Nucleic Acids Res. 23: 1269-70) or direct dideoxynucleotide sequencing, in which some or the entire DNA of interest that has been harvested from the sample is used as a template for sequencing reactions. An oligonucleotide primer or set of primers specific to the gene or DNA of interest is used in standard sequencing reactions. Other methods of DNA sequencing, such as sequencing by hybridization, sequencing using a "chip" containing many oligonucleotides for hybridization (as, for example, those produced by Affymetrix Corp.; Ramsay et al., 1998, Nature Biotechnology 16: 40-44; Marshall et al., 1998, Nature Biotechnology 16: 27-31), sequencing by HPLC (DeDionisio et al., 1996, J Chromatogr A 735: 191-208), and modifications of DNA sequencing strategies such as multiplex allele-specific diagnostic assay (MASDA; Shuber et al., 1997, Hum. Molec. Genet. 6: 337-47), dideoxy fingerprinting (Sarkar et al., 1992, Genomics 13: 441-3;; Martincic et al., 1996, Oncogene 13: 2039-44), and fluorogenic probe-based PCR methods (such as Taqman; Perkin-Elmer Corp.; Heid et al., 1996, Genome Res. 6: 986-94) and cleavase-based methods may be used.

Alternatively, amplification can be carried out using primers that are appropriately labelled, and the amplified primer extension products can be detected using procedures and equipment for detection of the label. Preferably probes of this invention are labeled with at least one detectable moiety, wherein the detectable moiety or moieties are selected from the group consisting of: a conjugate, a branched detection system, a chromophore, a fluorophore, a spin label, a radioisotope, an enzyme, a hapten, an acridinium ester and a luminescent compound. As an illustrative, non limitative, example, in the method of the present invention the primers used can labelled with a fluorophore. More particularly, the reverse primer of the method of the present invention is labelled with the 6-FAM fluorophore at its 5' end. This fluorophore emits fluorescence with a peak wavelength of 522 nm. The PCR can be carried out using one of the primers labelled with, for example, either FAM, HEX, VIC or NED dyes.

In a preferred embodiment of the invention, the posterior detection and analysis of the DNA amplified with the method of the invention is carried out by the GeneScan technique as it is illustrated in EP2046985. Thus, as an illustrative, non limitative, example for carrying out the detecting step of the method of the invention, an aliquot of the PCR reaction (typically 1 µl) is added to 9 µl of formamide HI-DI and 0.25 µl of GeneScan marker -500 LIZ size standard. After denaturation, the sample is placed in the ABI 3130 Genetic Analyzer and capillary electrophoresis is carried out. The raw data is analysed using GeneScan software. This analysis is very important since the PCR products will be sized by extrapolation to an in-sample size standard. Using this technique inventors are able to detect in a very precise and accurate manner the mutation of interest.

Independently of the method used for detecting mutated DNA, the number of copies of the nucleic acid sequence of the at least one sensitivity mutation of the EGFR gene towards an inhibitor of EGFR tyrosine kinase activity can be determined using calibration curves. The term "calibration curve" refers to a curve made using the same methods for detecting the mutated DNA using a sample with a defined number of copies of said mutated DNA. Thus, the results obtained from the sample of the patient can be compared to the curve determining the corresponding number of copies of said mutated DNA.

The number of copies of the non mutated EGFR gene can be determined using similar methods as described for the determination of the number of copies of the mutated sequence. As the person skilled in the art will understand, the probes used for determining the number of copies o the non mutated EGFR gen in the sample are adapted to detecting the sequence of the non mutated EGFR gene.

In a particular embodiment of the invention, the serum or plasma may be utilized directly for identification and quantification of the mutant DNA. In another particular embodiment, nucleic acid is extracted from plasma or serum as an initial step of the invention. In such cases, the total DNA extracted from said samples would represent the working material suitable for subsequent amplification.

Once the sample has been obtained, amplification of nucleic acid is carried out. In a particular embodiment, the amplification of the DNA is carried out by means of PCR. The general principles and conditions for amplification and detection of nucleic acids, such as using PCR, are well known for the skilled person in the art. In particular, the Polymerase Chain Reaction (PCR) carried out by the method of the present invention uses appropriate and specific oligonucleotide primers or amplification oligonucleotides to specifically amplify the EGFR target sequences. The terms "oligonucleotide primers" or "amplification oligonucleotides" are herein used indistinguishably and refer to a polymeric nucleic acid having generally less than 1,000 residues, including those in a size range having a lower limit of about 2 to 5 residues and an upper limit of about 500 to 900 residues. In preferred embodiments, oligonucleotide primers are in a size range having a lower limit of about 5 to about 15 residues and an upper limit of about 100 to 200 residues. More preferably, oligonucleotide primers of the present invention are in a size range having a lower limit of about 10 to about 15 residues and an upper limit of about 17 to 100 residues. Although oligonucleotide primers may be purified from naturally occurring nucleic acids, they are generally synthesized using any of a variety of well known enzymatic or chemical methods. In a particular embodiment of the invention, such oligonucleotide primers enable the specific amplification of the DNA fragments corresponding to the deletion of specific nucleotides in the exon 19 at the EGFR gene.

Thus, in a particular embodiment, the method of the invention can be used for the detection of ELREA deletions at the exon 19. In a preferred embodiment, the present invention refers to a method for the detection of 9, 12, 15, 18, or 24 nucleotides deletions in the exon 19 at the EGFR gene.

In another particular embodiment, the method of the invention can be used for the detection of the L858R mutation at the exon 21 of the EGFR gene. In another embodiment, the method of the invention can be used for the detection of the T790M mutations in exon 21 of the EGFR gene.

The term "amplification oligonucleotide" refers to an oligonucleotide that hybridizes to a target nucleic acid, or its complement, and participates in a nucleic acid amplification reaction. Amplification oligonucleotides include primers and promoter-primers in which the 3' end of the oligonucleotide is extended enzymatically using another nucleic acid strand as the template. In some embodiments, an amplification oligonucleotide contains at least about 10 contiguous bases, and more preferably about 12 contiguous bases, that are complementary to a region of the target sequence (or its complementary strand). Target-binding bases are preferably at least about 80%, and more preferably about 90% to 100% complementary to the sequence to which it binds. An amplification oligonucleotide is preferably about 10 to about 60 bases long and may include modified nucleotides or base analogues.

The terms "amplify" or "amplification" refer to a procedure to produce multiple copies of a target nucleic acid sequence or its complement or fragments thereof (i.e., the amplified product may contain less than the complete target sequence). For example, fragments may be produced by amplifying a portion of the target nucleic acid by using an amplification oligonucleotide which hybridizes to, and initiates polymerization from, an internal position of the target nucleic acid. Known amplification methods include, for example, polymerase chain reaction (PCR) amplification, replicase-mediated amplification, ligase chain reaction (LCR) amplification, strand-displacement amplification (SDA) and transcription-associated or transcription-mediated amplification (TMA). PCR amplification uses DNA polymerase, primers for opposite strands and thermal cycling to synthesize multiple copies of DNA or cDNA. Replicase-mediated amplification uses QB-replicase to amplify RNA sequences. LCR amplification uses at least four different oligonucleotides to amplify complementary strands of a target by using cycles of hybridization, ligation, and denaturation. SDA uses a primer that contains a recognition site for a restriction endonuclease and an endonuclease that nicks one strand of a hemimodified DNA duplex that includes the target sequence, followed by a series of primer extension and strand displacement steps. An isothermal strand-displacement amplification method that does not rely on endonuclease nicking is also known. Transcription-associated or transcription-mediated amplification uses a primer that includes a promoter sequence and an RNA polymerase specific for the promoter to produce multiple transcripts from a target sequence, thus amplifying the target sequence.

Preferred embodiments of the present invention amplify the EGFR target sequences using the present amplification oligonucleotides in a polymerase chain reaction (PCR) amplification. One skilled in the art will appreciate that these amplification oligonucleotides can readily be used in other methods of nucleic acid amplification that uses polymerase-mediated primer extension.

Methods for detecting mutations in the tyrosine kinase domain of the EGF receptor are known in the art, several corresponding diagnostic tools are approved by the FDA and commercially available, e.g. an assay for the detection of epidermal growth factor receptor mutations in patients with non-small cell lung cancer (Genzyme Corp.; see also Journal of Clinical Oncology, 2006 ASCO Annual Meeting Proceedings (Post-Meeting Edition). VoI 24, No 18S (June 20 Supplement), 2006: Abstract 10060). In a preferred embodiment, the mutations in EGFR are determined in serum samples as described in WO07039705 based on the use of specific Scorpion probes in combination with the Amplification Refractory Mutation System (ARMS) (Nucleic Acids Res., 1989, 17:2503-2516 and Nature Biotechnology, 1999, 17:804-807).

In a preferred embodiment, the number of copies of nucleic acid of the EGFR gene carrying at least one sensitivity mutation of the EGFR is measured by a method comprising the steps of
(i) amplifying the nucleic acid sequence corresponding to said specific region of the sensitivity mutation of the EGFR gene by means of PCR using a Protein-Nucleic Acid probe, wherein said Protein-Nucleic Acid probe is capable of specifically recognising and hybridising with the EGFR wild type sequence thereby inhibiting its amplification and
(ii) quantifying the number of copies of the nucleic acid sequence of the at least one sensitivity mutation of the EGFR gene.

In a still more preferred embodiment, the Protein-Nucleic Acid probe which is capable of specifically recognising and hybridising with the EGFR wild type sequence thereby inhibiting its amplification has a sequence selected from the group consisting of SEQ ID NO:3 (for detecting ELREA deletions in exon 19) and SEQ ID NO:10 (for detecting the L858R mutation in exon 21) such as it is described in WO08009740.

In the amplifying step, the nucleic acid sequence corresponding to a specific region of the EGFR gene is amplified by means of PCR using a Protein-Nucleic Acid (PNA) probe. PNA probes are nucleic acid analogs in which the sugar phosphate backbone of a natural nucleic acid has been replaced by a synthetic peptide backbone, usually formed from N-(2-aminoethyl)-glycine units, resulting in an achiral and uncharged mimic. This new molecule is chemically stable and resistant to hydrolytic (enzymatic) cleavage and thus not expected to be degraded inside a living cell. Despite all these variations from natural nucleic acids, PNA is still capable of sequence-specific binding to DNA as well as RNA obeying the Watson-Crick hydrogen bonding rules. Its hybrid complexes exhibit extraordinary thermal stability and display unique ionic strength properties. In many applications, PNA probes are preferred to nucleic acid probes because, unlike nucleic acid/nucleic acid duplexes which are destabilized under conditions of low salt, PNA/nucleic acid duplexes are formed and remain stable under conditions of very low salt. Those of ordinary skill in the art of nucleic acid hybridization will recognize that factors commonly used to impose or control stringency of hybridization include formamide concentration (or other chemical denaturant reagent), salt concentration (i.e., ionic strength), hybridization temperature, detergent concentration, pH and the presence or absence of chaotropes. Optimal stringency for a probe/target sequence combination is often found by the well known technique of fixing several of the aforementioned stringency factors and then determining the effect of varying a single stringency factor. The same stringency factors can be modulated to thereby control the stringency of hybridization of a PNA to a nucleic acid, except that the hybridization of a PNA is fairly independent of ionic strength. Optimal stringency for an assay may be experimentally determined by examination of each stringency factor until the desired degree of discrimination is achieved.

PNA oligomers can be prepared following standard solid-phase synthesis protocols for peptides (Merrifield, B. 1986. Solid-phase synthesis. Science 232, 341-347) using, for example, a (methyl-benzhydryl)amine polystyrene resin as the solid support. PNAs may contain a chimeric architecture, such as a PNA/DNA chimera, where a PNA oligomer is fused to a DNA oligomer.

Clinical samples contain DNA molecules with the wild-type allele in addition to DNA molecules with the mutant allele. So, under normal conditions, it is difficult to detect EGFR mutations (mutant allele) in a large background of wild-type EGFR genes (wild-type allele). In a particular case, the PNA probe utilized by the inventors is capable of specifically recognize and hybridize with the wild-type EGFR sequence. As an illustrative, non limitative example, the PNA probe to be used for carrying out the method of the present invention comprises the PNA probe described as the SEQ ID NO:3 or SEQ ID NO:10 in the Example accompanying the present invention. Such probe is added to the PCR reaction mix thus inhibiting amplification of the wild-type allele and favouring amplification of the mutant allele present in the sample, i.e. EGFR mutant, facilitating its posterior detection. Those of ordinary skill in the art will appreciate that a suitable PNA probe do not need to have exactly these probing nucleic acid sequences to be operative but often modified according to the particular assay conditions. For example, shorter PNA probes can be prepared by truncation of the nucleic acid sequence if the stability of the hybrid needs to be modified to thereby lower the Tm and/or adjust for stringency. Similarly, the nucleic acid sequence may be truncated at one end and extended at the other end as long as the discriminating nucleic acid sequence remains within the sequence of the PNA probe. Such variations of the probing nucleic acid sequences within the parameters described herein are considered to be embodiments of this invention.

The term "ratio between the number of copies of the nucleic acid sequence of the at least one sensitivity mutation of the EGFR gene towards an inhibitor of EGFR tyrosin kinase activity and the number of copies of the nucleic acid sequence of the non mutated EGFR gene" refers to relationship (division) between the number of copies of the nucleic acid sequence of the at least one sensitivity mutation of the EGFR gene towards an inhibitor of EGFR tyrosin kinase activity and the number of copies of the nucleic acid sequence of the non mutated EGFR gene.

The term "non mutated EGFR gene" refers to the gene that codifies the EGFR protein or a functional variant thereof. Preferably, the EGFR gene is the human EGFR gene.

Methods for determining whether a given mutant confers sensitivity to a tyrosine kinase activity have been described in detail in the prior art and include, among others, a method as described in WO2006091889 based on the detection of the autophosphorylation capacity of EGFR as measured in cells over-expressing EGFR in response to the treatment with a gefintib (Iressa™) or panitumumab.

In a second step, the method of the invention involves determining at a second time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid of the non mutated EGFR gene.

The determination of the ratio of copies of the nucleic acid sequence of the EGFR gene which contains at least one mutation conferring sensitivity of EGFR towards an inhibitor of EGFR tyrosine kinase activity and the number of copies of the nucleic acid of the non mutated EGFR gene is determined essentially as done in the first step.

The expression "the second time point is later than the first time point", as used herein, refers to the determination of the ratios of mutated vs. wild-type gene for a given sensitivity mutation at different moments during the monitoring of the patient. Preferably, the difference in time between the first and the second measurement is of 1 day, 2 days, 3 days, 4 days, 5 days, 6 days, one week, two weeks, three weeks, four weeks, 1 month, 2 months, 3 months, 4 months, 5 months, 6 months, 7 months, 8 months, 9 months, 10 months, 11 months, 1 year, 2 years or more.

In a preferred embodiment, the patient to which the method is applied does not show relapse symptoms or signs at the time point wherein the first measurement is made and/or at the time point whether the second measurement is made. The term "does not show relapse symptoms or signs", as used herein, refers to the absence of one or more of the clinical or biochemical features of the disease including, without limitation, cough, pain, abnormalities in the chest radiograph or computer tomography such as obvious mass, widening of the mediastinum, atelectasis (collapse), consolidation (pneumonia), no evidence of disease by Imaging DATA (IE PET scan) or pleural effusion, etc.

Once the ratios between number of mutated and number of wild-type genes has been determined, the method of the invention further comprises comparing said ratios wherein if the ratio determined at the second time point is higher than the ratio determined at the first time point, then it is indicative of a negative clinical response and wherein a decrease in the ratio determined at the second time point with respect to the ratio determined at the first time point is indicative of a positive clinical response of said patient to said EGFR inhibitor-based therapy.

The expression "positive response" when referred to the treatment with an EGFR tyrosine kinase inhibitor, as used herein, refers to any response which is substantially better than that obtained with a saline control or placebo.

The expression "negative response" when referred to the treatment with an EGFR tyrosine kinase inhibitor, as used herein, refers to any response which is substantially worse than that obtained with a saline control or placebo.

The response (positive or negative) can be assessed using any endpoint indicating a benefit to the patient, including, without limitation, (1) inhibition, to some extent, of tumor growth, including slowing down and complete growth arrest; (2) reduction in the number of tumor cells; (3) reduction in tumor size; (4) inhibition (i.e., reduction, slowing down or complete stopping) of tumor cell infiltration into adjacent peripheral organs and/or tissues; (5) inhibition of metastasis; (6) enhancement of anti-tumor immune response, possibly resulting in regression or rejection of the tumor; (7) relief, to some extent, of one or more symptoms associated with the tumor; (8) increase in the length of survival following treatment; and/or (9) decreased mortality at a given point of time following treatment.

The clinical response may also be expressed in terms of various measures of clinical outcome. Positive clinical outcome can also be considered in the context of an individual's outcome relative to an outcome of a population of patients having a comparable clinical diagnosis, and can be assessed using various endpoints such as an increase in the duration of Recurrence-Free interval (RFI), an increase in the time of survival as compared to Overall Survival (OS) in a population, an increase in the time of Disease-Free Survival (DFS), an increase in the duration of Distant Recurrence-Free Interval (DRFI), and the like. An increase in the likelihood of positive clinical response corresponds to a decrease in the likelihood of cancer recurrence.

The response in individual patients may be characterized as a complete response, a partial response, stable disease, and progressive disease, as these terms are understood in the art. In certain embodiments, the response is a pathological complete response. A pathological complete response, e.g., as determined by a pathologist following examination of tissue removed at the time of surgery or biopsy, generally refers to an absence of histological evidence of invasive tumor cells in the surgical specimen.

The positive or negative response can also be determined using the markers known to the skilled person. Suitable markers for determining whether a patient has had a positive response include, without limitation, the criteria described by WHO (Miller AB. et al., 1981, Cancer 47:207-214) as well as the criteria of the Response Evaluation Criteria In Solid Tumors (RECIST) as described by Therasse P. et al. (J. Natl. Cancer Inst., 2000; 92:205-216) and by Eisenhauer et al. (European Journal of Cancer, 2009, 45:228 -247), including
- *Complete Response (CR):* Disappearance of all target lesions
- *Partial Response (PR):* At least a 30% decrease in the sum of the longest diameter (LD) of target lesions, taking as reference the baseline sum LD
- *Stable Disease (SD):* Neither sufficient shrinkage to qualify for PR nor sufficient increase to qualify for PD, taking as reference the smallest sum LD since the treatment started
- *Progressive Disease (PD):* At least a 20% increase in the sum of the LD of target lesions, taking as reference the smallest sum LD recorded since the treatment started or the appearance of one or more new lesions

The expression "a ratio at the first time point higher than the ratio at the second time point", as used herein, indicates that the value of the ratio at the second time point is statistically higher than the ratio at the first time point. Preferably, the ratio at the second time point is at least 10%, at least 20%, at least 30%, at least 40%, at least 50%, at least 60%, at least 70%, at least 80%, at least 90% or more than the ratio at the first time point.

The term "negative response" is understood as a situation where at least 10%, 20%, 30%, 40%, 50%, 60%, 70%, 80%, 90% of the patients have a negative result regarding the endpoint parameters described above.

Conversely, wherein in the method of the invention the ratio determined at the second time point is lower than the ratio determined at the first time point, then it is indicative of a positive clinical outcome. The expression "positive clinical outcome" is understood as a situation where at least 10%, 20%, 30%, 40%, 50%, 60%, 70%, 80%, 90% of the patients have a positive result regarding the endpoint parameters described above.

In a preferred embodiment, the method according to the invention further comprises determining at said first and second time points the ratios between the number of copies of the nucleic acid sequence of the EGFR gene which contains at least one mutation conferring resistance of EGFR towards an inhibitor of EGFR tyrosine kinase activity and the number of copies of the nucleic acid of the non mutated EGFR gene in the biofluid of the patient and wherein an increase in the ratio determined at the second time point with respect to the ratio at determined at the first time point is indicative of a negative clinical outcome.

The term "mutation conferring resistance of EGFR towards an inhibitor of EGFR tyrosine kinase activity", as used herein, refers to mutants in the tyrosine kinase domain of EGFR which result in an decreased inhibition of the tyrosine kinase activity of EGFR in response to the treatment with inhibitor such as erlotinib with respect to the wild-type protein. Mutations conferring resistance to EGFR TK inhibitors include, without limitation, exon 20 insertion mutants D770_N771 (ins NPG), D770_(ins SVQ) and D770_(ins G) N771T, the T790M mutation in exon 20 and the D761Y mutation. In a preferred embodiment, the resistance mutation of the EGFR gene towards an inhibitor of tyrosine kinase activity is the T790M mutation in exon 20.

Methods for the determination of the number of copies of nucleic acids carrying a resistance mutation in the EGFR gene and for determining the ratio of said genes to wild-type genes are essentially as described in respect to the sensitivity mutations. In a preferred embodiment, the determination of the number of copies of EGFR nucleic acids containing a mutation conferring resistance to EGFR TK inhibitors is carried out by RT-PCR in the presence of a PNA probe which binds is capable of specifically recognising and hybridising with the EGFR wild type sequence thereby inhibiting its amplification. In a preferred embodiment, the detection of mutations in the EGFR gene conferring resistance to an inhibitor of EGFR tyrosine kinase activity is carried out by
(i) amplifying the nucleic acid sequence corresponding to said specific region of the sensitivity mutation of the EGFR gene by means of PCR using a Protein-Nucleic Acid probe, wherein said Protein-Nucleic Acid probe is capable of specifically recognising and hybridising with the EGFR wild type sequence thereby inhibiting its amplification
(ii) quantifying the number of copies of the nucleic acid sequence of the at least one sensitivity mutation of the EGFR gene.

In a preferred embodiment, the resistance mutation of the EGFR gene towards an inhibitor of tyrosine kinase activity is the T790M mutation in exon 20. In a still more preferred embodiment, the presence of a resistance mutation in the EGFR gene resulting in an EGFR variant showing resistance towards an inhibitor of tyrosine kinase activity is determined by PCR amplification amplifying the nucleic acid sequence corresponding to said specific region of the sensitivity mutation of the EGFR gene by means of PCR using a Protein-Nucleic Acid probe, wherein said Protein-Nucleic Acid probe is capable of specifically recognising and hybridising with the EGFR wild type sequence thereby inhibiting its amplification followed by quantifying the number of copies of the nucleic acid sequence of the at least one sensitivity mutation of the EGFR gene.

In a still more preferred embodiment, the PNA probe used for detecting the T790M mutation comprises the sequence of SEQ ID NO:15.

### Therapeutic methods of the invention

The authors of the present invention have observed that, surprisingly, patients which have suffered lung cancer and which show a high probability of relapsing show an increased in the number of copies of EGFR gene in a biofluid carrying mutations conferring sensitivity to an EGFR tyrosine kinase inhibitor. This increase can be detected even when the patient remains asymptomatic and before the appearance of tumor related symptoms and/or imaging evidence of progression. Thus, these patients are candidates for the treatment with an EGFR tyrosine kinase inhibitor. Thus, in another aspect, the invention relates to a composition comprising an EGFR inhibitor for use in the treatment of lung cancer in a patient wherein said patient is selected by a method comprising
(i) determining at a first time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid sequence of the non mutated EGFR gene and
(ii) determining at a second time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid of the non mutated EGFR gene,
wherein said second time point is later than the first time point and
wherein the ratio determined at the second time point is increased with respect to the ratio at determined at the first time point.

The expressions "lung cancer", "positive response to an EGFR tyrosine kinase inhibitor-based chemotherapy", "patient", "first time point", "second time point", "biofluid", "mutation conferring sensitivity of EGFR towards an inhibitor of EGFR tyrosine kinase", "second time point being is later than the first time point" have been defined in respect of the first method of the invention and are equally applicable to the therapeutic method as described herein.

The term "treating" or its grammatical equivalents as used herein, means achieving a therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient may still be afflicted with the underlying disorder. For prophylactic benefit, the compositions may be administered to a patient at risk of developing a particular disease, or to a patient reporting one or more of the physiological symptoms of a disease, even though a diagnosis of this disease may not have been made.

The term "EGFR inhibitor" has been described in detail in the context of the first method of the invention. In a preferred embodiment, the EGFR inhibitor comprises a EGFR tyrosine-kinase inhibitor. In a still more preferred embodiment, the EGFR tyrosine kinase inhibitor is selected from the group of a dual EGFR inhibitor, a dual EGFR tyrosine kinase inhibitor or a EGFR tyrosine kinase inhibitor specific for EGFR carrying a resistance mutation. In a yet more preferred embodiment, the EGFR tyrosine-kinase inhibitor is erlotinib or gefitinib.

The tyrosine kinase inhibitors are then administered to patients as known in the art. The route of administration may oral, intravenous (LV.), intramuscular (LM.), subcutaneous (S.C.), intradermal (I.D.), intraperitoneal (LP.), intrathecal (LT.), intrapleural, intrauterine, rectal, vaginal, topical, intratumor and the like. The tyrosine kinase inhibitors can be administered parenterally by injection or by gradual infusion over time and can be delivered by peristaltic means.

Administration may be by transmucosal or transdermal means. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays, for example, or using suppositories.

For oral administration, the tyrosine kinase inhibitors are formulated into conventional oral administration forms such as capsules, tablets and tonics.

For topical administration, the pharmaceutical composition (inhibitor of kinase activity) is formulated into ointments, salves, gels, or creams, as is generally known in the art.

Typically, the tyrosine kinase inhibitors are administered orally by administration of a unit dose. The term "unit dose" when used in reference to a therapeutic composition of the present invention refers to physically discrete units suitable as unitary dosage for the subj ect, each unit containing a predetermined quantity of active material calculated to produce the desired therapeutic effect in association with the required diluent; i.e., carrier, or vehicle.

The compositions are administered in a manner compatible with the dosage formulation, and in a therapeutically effective amount. The quantity to be administered and timing depends on the subject to be treated, capacity of the subject's system to utilize the active ingredient, and degree of therapeutic effect desired. Precise amounts of active ingredient required to be administered depend on the judgment of the practitioner and are peculiar to each individual.

The tyrosine kinase inhibitors useful for practicing the methods of the present invention are described herein. Any formulation or drug delivery system containing the active ingredients, which is suitable for the intended use, as are generally known to those of skill in the art, can be used. Suitable pharmaceutically acceptable carriers for oral, rectal, topical or parenteral (including inhaled, subcutaneous, intraperitoneal, intramuscular and intravenous) administration are known to those of skill in the art. The carrier must be pharmaceutically acceptable in the sense of being compatible with the other ingredients of the formulation and not deleterious to the recipient thereof

As used herein, the terms "pharmaceutically acceptable", "physiologically tolerable" and grammatical variations thereof, as they refer to compositions, carriers, diluents and reagents, are used interchangeably and represent that the materials are capable of administration to or upon a mammal without the production of undesirable physiological effects.

Formulations suitable for parenteral administration conveniently include sterile aqueous preparation of the active compound which is preferably isotonic with the blood of the recipient. Thus, such formulations may conveniently contain distilled water, 5% dextrose in distilled water or saline. Useful formulations also include concentrated solutions or solids containing the compound which upon dilution with an appropriate solvent give a solution suitable for parenteral administration above.

For enteral administration, a compound can be incorporated into an inert carrier in discrete units such as capsules, cachets, tablets or lozenges, each containing a predetermined amount of the active compound; as a powder or granules; or a suspension or solution in an aqueous liquid or non-aqueous liquid, e.g., a syrup, an elixir, an emulsion or a draught. Suitable carriers may be starches or sugars and include lubricants, flavorings, binders, and other materials of the same nature.

A tablet may be made by compression or molding, optionally with one or more accessory ingredients. Compressed tablets may be prepared by compressing in a suitable machine the active compound in a free-flowing form, e.g., a powder or granules, optionally mixed with accessory ingredients, e.g., binders, lubricants, inert diluents, surface active or dispersing agents. Molded tablets may be made by molding in a suitable machine, a mixture of the powdered active compound with any suitable carrier.

A syrup or suspension may be made by adding the active compound to a concentrated, aqueous solution of a sugar, e.g., sucrose, to which may also be added any accessory ingredients. Such accessory ingredients may include flavoring, an agent to retard crystallization of the sugar or an agent to increase the solubility of any other ingredient, e.g., as a polyhydric alcohol, for example, glycerol or sorbitol.

Fomulations for rectal administration may be presented as a suppository with a conventional carrier, e.g., cocoa butter or Witepsol S55 (trademark of Dynamite Nobel Chemical, Germany), for a suppository base.

Formulations for oral administration may be presented with an enhancer. Orally-acceptable absorption enhancers include surfactants such as sodium lauryl sulfate, palmitoyl camitine, Laureth-9, phosphatidylcholine, cyclodextrin and derivatives thereof; bile salts such as sodium deoxycholate, sodium taurocholate, sodium glycochlate, and sodium fusidate; chelating agents including EDT A, citric acid and salicylates; and fatty acids (e.g., oleic acid, lauric acid, acylcamitines, mono and diglycerides). Other oral absorption enhancers include benzalkonium chloride, benzethonium chloride, CHAPS (3-(3-cholamidopropyl)-dimethylammoniolpropanesulfonate), Big-CHAPS (N, N-bis(3-D-gluconamidopropyl)-cholamide), chlorobutanol, octoxynol-9, benzyl alcohol, phenols, cresols, and alkyl alcohols. An especially preferred oral absorption enhancer for the present invention is sodium lauryl sulfate.

Alternatively, the tyrosine kinase inhibitor may be administered in liposomes or microspheres (or microparticles). Methods for preparing liposomes and microspheres for administration to a patient are well known to those of skill in the art. U.S. Pat. No. 4,789,734, the contents of which are hereby incorporated by reference, describes methods for encapsulating biological materials in liposomes. Essentially, the material is dissolved in an aqueous solution, the appropriate phospholipids and lipids added, along with surfactants if required, and the material dialyzed or sonicated, as. necessary. A review of known methods is provided by G. Gregoriadis, Chapter 14,"Liposomes," Drug Carriers in Biology and Medicine, pp. 287-341 (Academic Press,1979).

Microspheres formed of polymers or proteins are well known to those skilled.in the art, and can be tailored for passage through the gastrointestinal tract directly into the blood stream. Alternatively, the compound can be incorporated and the microspheres, or composite of microspheres, implanted for slow release over a period of time ranging from days to months. See, for example, U.S. Pat. Nos.4,906,474, and 4,925,673 and 3,625,214, and Jein, TIPS 19:155-157 (1998), the contents of which are hereby incorporated by reference.

In one embodiment, the tyrosine kinase inhibitor can be formulated into a liposome or microparticle which is suitably sized to lodge in capillary beds following intravenous administration. When the liposome or microparticle is lodged in the capillary beds surrounding ischemic tissue, the agents can be administered locally to the site at which they can be most effective. Suitable liposomes for targeting ischemic tissue are generally less than about 200 nanometers and are also typically unilamellar vesicles, as disclosed, for example, in U.S. Pat. No. 5,593,688 to Baldeschweiler, entitled "Liposomal targeting of ischemic tissue," the contents of which are hereby incorporated by reference.

Preferred microparticles are those prepared from biodegradable polymers, such as polyglycolide, polylactide and copolymers thereof. Those of skill in the art can readily determine an appropriate carrier system depending on various factors, including the desired rate of drug release and the desired dosage.

In one embodiment, the formulations are administered via catheter directly to the inside of blood vessels. The administration can occur, for example, through holes in the catheter. In those embodiments wherein the active compounds have a relatively long halflife (on the order of 1 day to a week or more), the formulations can be included in biodegradable polymeric hydrogels, such as those disclosed in U.S. Pat. No. 5,410,016 to Hubbell et al. These polymeric hydrogels can be delivered to the inside of a tissue lumen and the active compounds released over time as the polymer degrades. If desirable, the polymeric hydrogels can have microparticles or liposomes which include the active compound dispersed therein, providing another mechanism for the controlled release of the active compounds.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately bringing the active compound into association with a liquid carrier or a finely divided solid carrier and then, if necessary, shaping the product into desired unit dosage form.

The formulations may further include one or more optional accessory ingredient(s) utilized in the art of pharmaceutical formulations, e.g., diluents, buffers, flavoring agents, binders, surface active agents, thickeners, lubricants, suspending agents, preservatives (including antioxidants) and the like.

Compounds of the present methods may be presented for administration to the respiratory tract as a snuff or an aerosol or solution for a nebulizer, or as a micro fine powder for insufflation, alone or in combination with an inert carrier such as lactose. In such a case the particles of active compound suitably have diameters ofless than 50 microns, preferably less than 10 microns, more preferably between 2 and 5 microns.

Generally for nasal administration a mildly acid pH will be preferred. Preferably the compositions of the invention have a pH of from about 3 to 5, more preferably from about 3.5 to about 3.9 and most preferably 3.7. Adjustment of the pH is achieved by addition of an appropriate acid, such as hydrochloric acid.

The preparation of a pharmacological composition that contains active ingredients dissolved or dispersed therein is well understood in the art and need not be limited based on formulation. Typically such compositions are prepared as injectables either as liquid solutions or suspensions, however, solid forms suitable for solution, or suspensions, in liquid prior to use can also be prepared. The preparation can also be emulsified.

The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances such as wetting or emulsifying agents, pH buffering agents and the like which enhance the effectiveness of the active ingredient.

The tyrosine kinase inhibitor to be administered according to the present invention can include pharmaceutically acceptable salts of the components therein. Pharmaceutically acceptable salts include the acid addition salts (formed with the free amino groups of the polypeptide) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethyl amino ethanol, histidine, procaine and the like.

Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, polyethylene glycol and other solutes.

Liquid compositions can also contain liquid phases in addition to and to the exclusion of water. Exemplary of such additional liquid phases are glycerin, vegetable oils such as cottonseed oil, and water-oil emulsions.

If the tyrosine kinase inhibitor is based on RNA interference (e.g, an siRNA), the siRNAs may be chemically synthesized, produced using in vitro transcription, etc. In addition, the siRNA molecule can be customized to individual patients in such a way as to correspond precisely to the mutation identified in their tumor. Since siRNA can discriminate between nucleotide sequences that differ by only a single nucleotide, it is possible to design siRNAs that uniquely target a mutant form of the. EGFR gene that is associated with either a single nucleotide substitution or a small deletion of several nucleotides-both of which have been identified in tumors as described herein.

The delivery of siRNA to tumors can potentially be achieved via any of several gene delivery "vehicles" that are currently available. These include viral vectors, such as adenovirus, lentivirus, herpes simplex virus, vaccinia virus, and retrovirus, as well as chemical-mediated gene delivery systems (for example, liposomes), or mechanical DNA delivery systems (DNA guns). The oligonucleotides to be expressed for such siRNA-mediated inhibition of gene expression would be between 18 and 28 nucleotides in length.

In a preferred embodiment, the composition for use according to the invention is administered to patients wherein the selection additionally involves determining in a biofluid of the patient the ratio between the number of copies of the nucleic acid sequence of the EGFR gene which contains at least one mutation conferring resistance of EGFR towards an inhibitor of EGFR tyrosine kinase activity and the number of copies of the nucleic acid of the non mutated EGFR gene in the biofluid of the patient and wherein the patient is further selected wherein the wherein an increase in the ratio determined at the second time point is increased with respect to the ratio at determined at the first time point is indicative of a negative clinical outcome. In a still more preferred embodiment the resistance mutation of the EGFR gene towards an inhibitor of tyrosine kinase activity is the T790M mutation in exon 20.

In a preferred embodiment, the patient has suffered advanced lung cancer. In a still more preferred embodiment, the lung cancer is Non Small Cell Lung Cancer.

In a preferred embodiment, the composition for use according to the invention is administered to patients selected on the basis of the presence in atumor sample from said patient of a mutation in the EGFR gene which results in a EGFR showing resistance to an inhibitor of tyrosine kinase activity.

In a preferred embodiment, the patient which had suffered lung cancer has had surgery in addition to the EGFR tyrosine kinase inhibitor-based chemotherapy.

In another preferred embodiment, the biofluid is serum.

In another preferred embodiment, the the sensitivity mutation of the EGFR gene towards an inhibitor of tyrosine kinase activity, is selected from L858R mutation and an (E)LREA deletion in exon 19.

In another preferred embodiment, the composition for use according to the invention is administered to the patient which has no relapse symptoms at the first and/or second time points. In a still more preferred embodiment, the relapse symptoms are cough, pain or tumoral mass observed by PET/CT.

### Further aspects

[1] A method for predicting the clinical response of a patient suffering lung cancer to an EGFR inhibitor-based therapy comprising
   (i) determining at a first time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid sequence of the non mutated EGFR gene and
   (ii) determining at a second time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid of the non mutated EGFR gene,
   wherein said second time point is later than the first time point and
   wherein an increase in the ratio determined at the second time point with respect to the ratio determined at the first time point is indicative of a negative clinical response of said patient to said EGFR inhibitor-based therapy or
   wherein a decrease in the ratio determined at the second time point with respect to the ratio at determined at the first time point is indicative of a positive clinical response of said patient to said EGFR inhibitor-based therapy
[2] A method as defined in [1] further comprising determining at said first and second time points the ratio between the number of copies of the nucleic acid sequence of the EGFR gene which contains at least one mutation conferring resistance of EGFR towards an inhibitor of EGFR tyrosine kinase activity and the number of copies of the nucleic acid of the non mutated EGFR gene in the biofluid of the patient
   wherein an increase in the ratio determined at the second time point with respect to the ratio at determined at the first time point is indicative of a negative response or wherein a decrease in the ratio determined at the second time point with respect to the ratio at determined at the first time point is indicative of a positive response.
[3] A method as defined in [2] wherein the resistance mutation of the EGFR gene towards an inhibitor of tyrosine kinase activity is the T790M mutation in exon 20.
[4] A method as defined in any of [1] to [3], wherein the patient has advanced lung cancer.
[5] A method according to any of the preceding aspects, wherein the lung cancer is Non Small Cell Lung Cancer.
[6] A method according to any of the preceding aspects wherein the patient had been treated by surgery prior to the first time point.
[7] A method according to any of the preceding aspects, wherein a tissue sample from the tumor of the patient at the first time point is positive for a sensitivity mutation of the EGFR gene towards an inhibitor of tyrosine kinase activity.
[8] A method according to any of the preceding aspects, wherein the EGFR inhibitor-based therapy started prior to the first time point or between the first and the second time point.
[9] A method as defined in any of the preceding aspects, wherein the biofluid is serum.
[10] A method as defined in any of the preceding aspects, wherein the EGFR inhibitor-based therapy comprises a EGFR tyrosine-kinase inhibitor.
[11] A method as defined in [10] wherein the EGFR tyrosine kinase inhibitor is a dual EGFR inhibitor, a dual EGFR tyrosine kinase inhibitor or a EGFR tyrosine kinase inhibitor specific for EGFR carrying a resistance mutation.
[12] A method as defined in [11] wherein EGFR tyrosine-kinase inhibitor is erlotinib or gefitinib.
[13] A method as defined in any of the preceding aspects, wherein the sensitivity mutation of the EGFR gene towards an inhibitor of tyrosine kinase activity, is selected from L858R mutation and an (E)LREA deletion in exon 19.
[14] A method as defined in any of the preceding aspects, wherein the number of copies of nucleic acid of the EGFR gene carrying at least one sensitivity mutation of the EGFR is measured by
   (i) amplifying the nucleic acid sequence corresponding to said specific region of the sensitivity mutation of the EGFR gene by means of PCR using a Protein-Nucleic Acid probe, wherein said Protein-Nucleic Acid probe is capable of specifically recognising and hybridising with the EGFR wild type sequence thereby inhibiting its amplification
   (ii) quantifying the number of copies of the nucleic acid sequence of the at least one sensitivity mutation of the EGFR gene.
[15] A method as defined in any of the preceding aspects, wherein the clinical response is determined as progression free survival, response, survival or relapse.
[16] A method as defined in any of the preceding aspects, wherein the patient shows no relapse symptoms between the first and the second time points.
[17] A method as defined in [16] wherein said relapse symptoms are cough, pain or tumoral mass observed by PET/CT.
[18] A composition comprising an EGFR inhibitor for use in the treatment of lung cancer in a patient suffering lung cancer wherein said patient is selected by a method comprising
   (i) determining at a first time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid sequence of the non mutated EGFR gene and
   (ii) determining at a second time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid of the non mutated EGFR gene,
   wherein said second time point is later than the first time point and
   wherein the ratio determined at the second time point is increased with respect to the ratio at determined at the first time point.
[19] A composition for use as defined in [18] wherein the method for selecting the patient further comprises determining at said first and second time points the ratio between the number of copies of the nucleic acid sequence of the EGFR gene which contains at least one mutation conferring resistance of EGFR towards an inhibitor of EGFR tyrosine kinase activity and the number of copies of the nucleic acid of the non mutated EGFR gene in the biofluid of the patient and wherein the patient is further selected wherein an increase in the ratio determined at the second time point with respect to the ratio determined at the first time is determined.
[20] A composition for use according to [19] wherein the resistance mutation of the EGFR gene towards an inhibitor of tyrosine kinase activity is the T790M mutation in exon 20.
[21] A composition for use according to any of [18] to [20] wherein lung cancer is advanced lung cancer.
[22] A composition for use according to any of [18] to [20] wherein the lung cancer is Non Small Cell Lung Cancer.
[23] A composition for use according to any of [18] to [20] wherein the method further comprises selecting those patients wherein a tissue sample from the tumor at the first time point is positive for a sensitivity mutation of the EGFR gene towards an inhibitor of tyrosine kinase activity.
[24] A composition for use according to any of [18] to [23] wherein the biofluid is serum.
[25] A composition for use according to any of [18] to [24] wherein the EGFR inhibitor-based therapy comprises a EGFR tyrosine-kinase inhibitor.
[26] A composition for use according to [25] wherein the EGFR tyrosine kinase inhibitor is a dual EGFR inhibitor, a dual EGFR tyrosine kinase inhibitor or a EGFR tyrosine kinase inhibitor specific for EGFR carrying a resistance mutation.
[27] A composition for use according to [26] wherein the EGFR tyrosine-kinase inhibitor is erlotinib or gefitinib.
[28] A composition for use according to any of [18] to [27] wherein the sensitivity mutation of the EGFR gene towards an inhibitor of tyrosine kinase activity, is selected from L858R mutation and an (E)LREA deletion in exon 19.
[29] A composition for use according to any of [18] to [28] wherein the patient has no relapse symptoms at the first and/or second time points.
[30] A method as defined in claim [29] wherein said relapse symptoms are cough, pain or tumoral mass observed by PET/CT.

The invention is described in detail by way of the following examples which are to be considered as merely illustrative and not limitative of the scope of the invention.

### EXAMPLES

### METHODS: MONITORING EGFR MUTATIONS IN SERUM

### Blood samples

Blood (15 mL) was collected from patients in three Vacutainer tubes (Becton Dickinson, Plymouth, UK), two for serum and one for plasma. Tubes were centrifuged twice at 2300 rpm for 10 min and the supernatant (serum or plasma) alliquoted. DNA was purified from 0.4 mL of serum or plasma by standard procedures, using the QIAamp DNA Blood Mini Kit (Qiagen), and resuspended in 20 µL of water. For each patient, DNA extraction and mutation analysis was performed per quadruplicate in two samples of serum and two samples of plasma. DNA from the cell line PC-9 was used as a mutated control for exon 19, and wild-type control for exons 20 and 21. DNA from the H1975 cell line was used as a wild-type control for exon 19, and mutated control for exons 20 and 21.

### Nested length analysis of fluorescently labelled PCR products for EGFR deletions in exon 19

For the first PCR, primers were as follows: forward 5'-GTGCATCGCTGGTAACATCC-3' (SEQ ID NO: 1) and reverse 5'-TGTGGAGATGAGCAGGGTCT- 3' (SEQ ID NO: 2 ). Peptide Nucleic Acid (PNA): 5'-AGATGTTGCTTCTCTTA-3' (SEQ ID NO: 3). The first PCR was performed in 25-µl volumes adding 2 µl of sample, 0.125 µl of Ecotaq Polymerase (Ecogen, Barcelona, Spain), 2,5 µL of PCR buffer x10, 0,625 µL dNTPs (10 mM), 0,75 µL MgCl2 (50 mM), 1.25 pmol of each primer (10 µM) and 12,5 µL of 10 mM PNA probe. Amplification was as follows: 25 cycles of 30 seconds at 94°C, 30 seconds at 64°C, and 1 minute at 72°C (exons 19 and 21), or 35 cycles of 30 seconds at 94°C, 30 seconds at 58°C, and 1 minute at 72°C (exon 20).

For the length analysis, amplification was performed with the following primers: forward 5'-ACTCTGGATCCCAGAAGGTGAG-3' (SEQ ID NO:4) and reverse 5'-FAM-CCACACAGCAAAGCAGAAACTC-3' (SEQ ID NO: 5). Amplification (35 cycles) was done for 30 seconds at 94°C, 30 seconds at 58°C, and 1 minute at 72°C in 25-µl volumes adding 2 µl of sample, 0.1 µl of Ecotaq Polymerase (Ecogen, Barcelona, Spain), 2,5 µL of PCR buffer x10, 0,625 µL dNTPs (10 mM), 1 µL MgCl2 (50 mM), 1.25 pmol of each primer (10 µM) and 7,5 µL of 10 mM PNA probe. One microliter of a 1/200 dilution of each PCR product was mixed with 0.5 µl of size standard (Applied Biosystems) and denatured in 9 µl formamide at 90°C for 5 minutes. Separation was done with a four-color laser-induced fluorescence capillary electrophoresis system (ABI Prism 3130 Genetic Analyzer, Applied Biosystems). The collected data were evaluated with the GeneScan Analysis Software (Applera, Norwalk, CT).

### TaqMan assay for EGFR mutation in exons 20 (T790M) and 21 (L858R)

Primers and probes were as follows: exon 21 (forward primer, 5'-AACACCGCAGCATGTCAAGA-3' (SEQ ID NO: 6), reverse primer 5'-TTCTCTTCCGCACCCAGC-3' (SEQ ID NO: 7); probes 5'-FAM-CAGATTTTGGGCGGGCCAAAC-TAMRA-3' (SEQ ID NO: 8); and 5'-VIC-TCACAGATTTTGGGCTGGCCAAAC-TAMRA-3' (SEQ ID NO: 9), PNA: AGTTTGGCCAGCCCA (SEQ ID NO: 10) and exon 20 (forward primer, 5'-AGGCAGCCGAAGGGCA-3' (SEQ ID NO: 11), reverse primer 5'-CCTCACCTCCACCGTGCA-3' (SEQ ID NO: 12); probes 5' VIC-CTCATCACGCAGCTCATG -MGB-3' (SEQ ID NO: 13); and 5'-FAM-CTCATCATGCAGCTCATG - MGB-3' (SEQ ID NO: 14), PNA: TCATCACGCAGCTC (SEQ ID NO: 15)). Amplification was performed in 12.5-µl volumes using 1 of sample, 6.25 µl of Ampli Taq Gold PCR Master Mix (Applied Biosystems), 0.75 µl of each primer (10 µM), 0.25 µL of probes (10 µM) and 0,625 µl of PNA (10 µM). Samples were submitted to 50 cycles of 15 seconds at 94°C and 1 minute at 60°C in an Applied Biosystems 7000 real-time cycler.

### Calculations

For exon 19, a sample is considered positive (mutation detected) if a peak of mutated allele appears at least in one of the alliquots analyzed. The number of alliquots showing a mutated peak is recorded. In addition, another indicator is calculated as follows: area of the mutated peaks (in the four alliquots) / total area of the wt + mutated peaks (also in the four alliquots)

For exon 20, a sample is considered positive (mutation detected) if at least in one of the alliquots analyzed is positive. The number of alliquots were the mutation is detected is recorded.

### Results

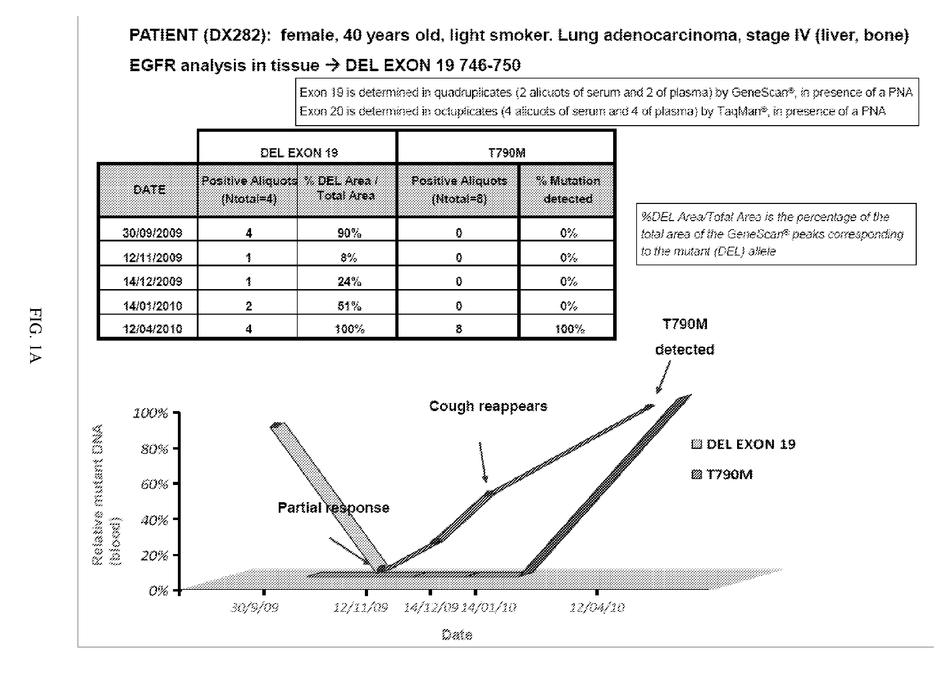

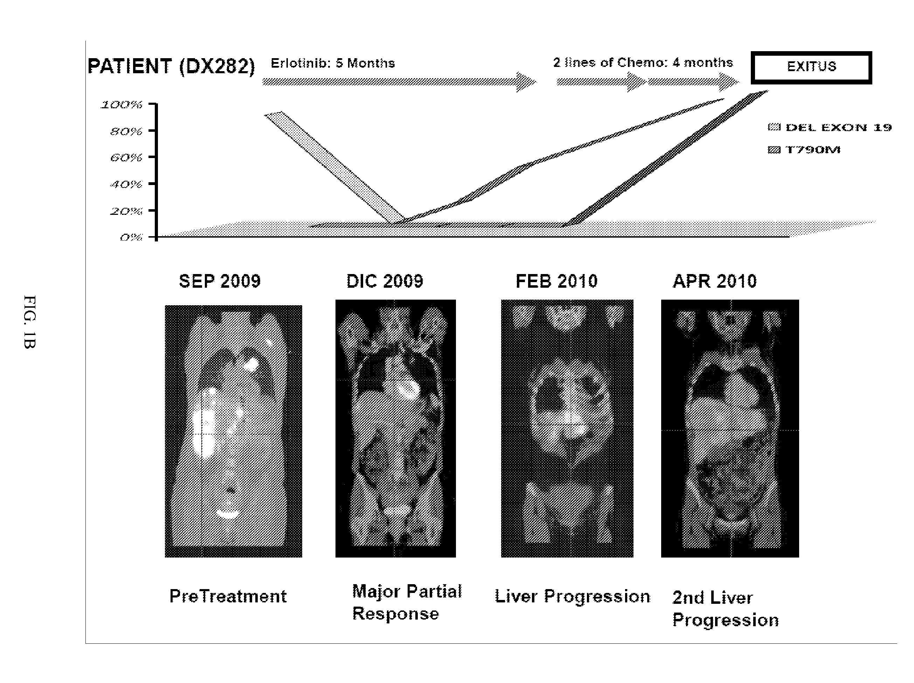

Patient DX 282 (Fig. 1): The dynamics (decrease) of the sensitive mutation in serum predicts the onset of radiological and clinical response. The increase in the ratios of the sensitive mutations (DEL EXON 19) anticipates and correlates with the emergence of symptoms before the confirmation of liver progression by CT scan. There is a late appearance of the resistance mutation while on second and third line chemotherapy.

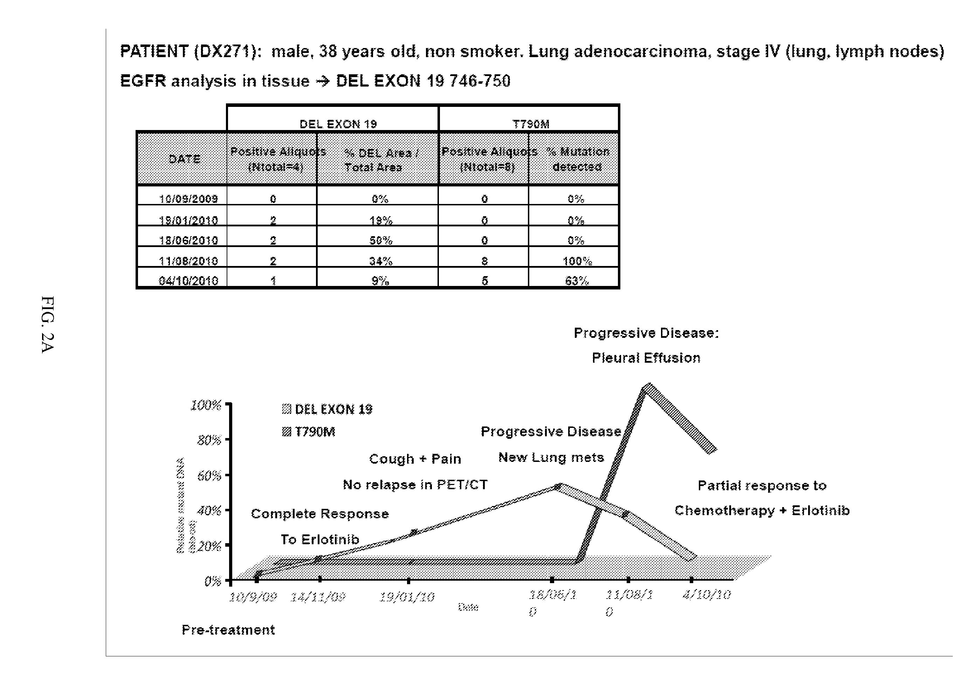

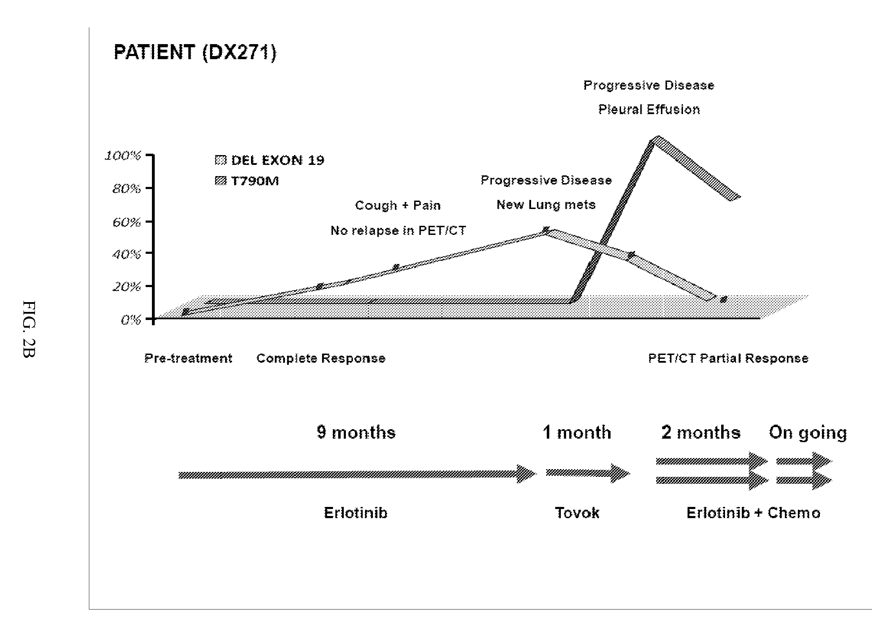

Patient DX271 (Fig. 2); The dynamics (increase) of the sensitive mutation on T2 and T3 correlates with the appearance of new symptoms before the radiological evidence of progressive disease. Moreover this patient was treated with Tovok manifesting a drop in the sensitive mutation but the emergence of the resistant mutation in line with the onset of progressive disease to the agent. Both the EGFR sensitive and resistant mutation are going down in line with a new clinical response to CDDP+Erlotinib.

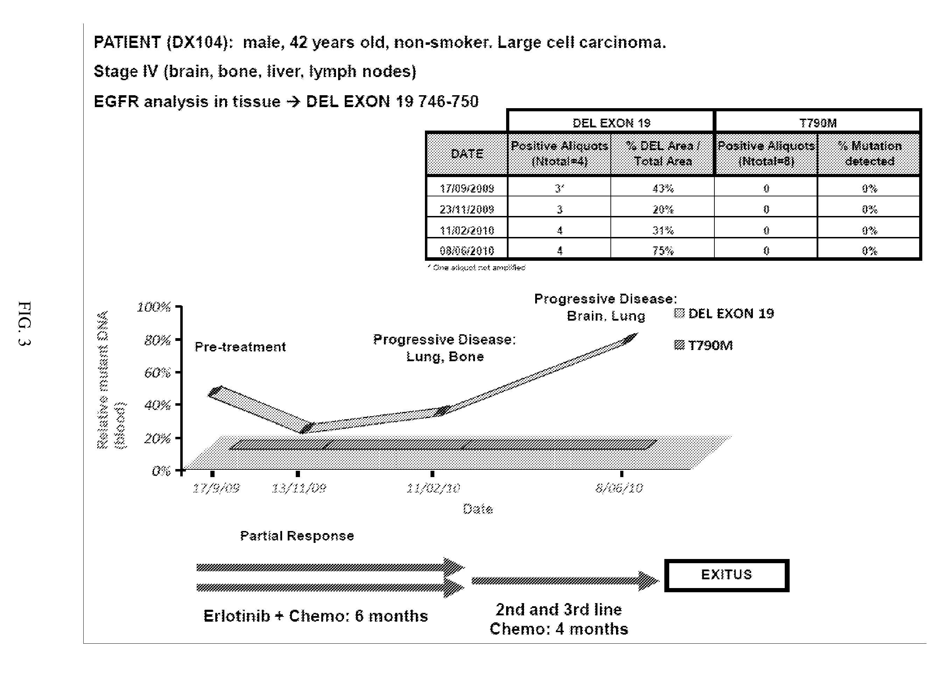

Patient DX 104 (Fig. 3): In this case the dynamics of the EGFR mutation levels in serum correlates with the initial short lasting response and anticipate the onset of progressive disease; note that the rations increase while on 2 and third line chemotherapy, the resistant mutation was never detected.

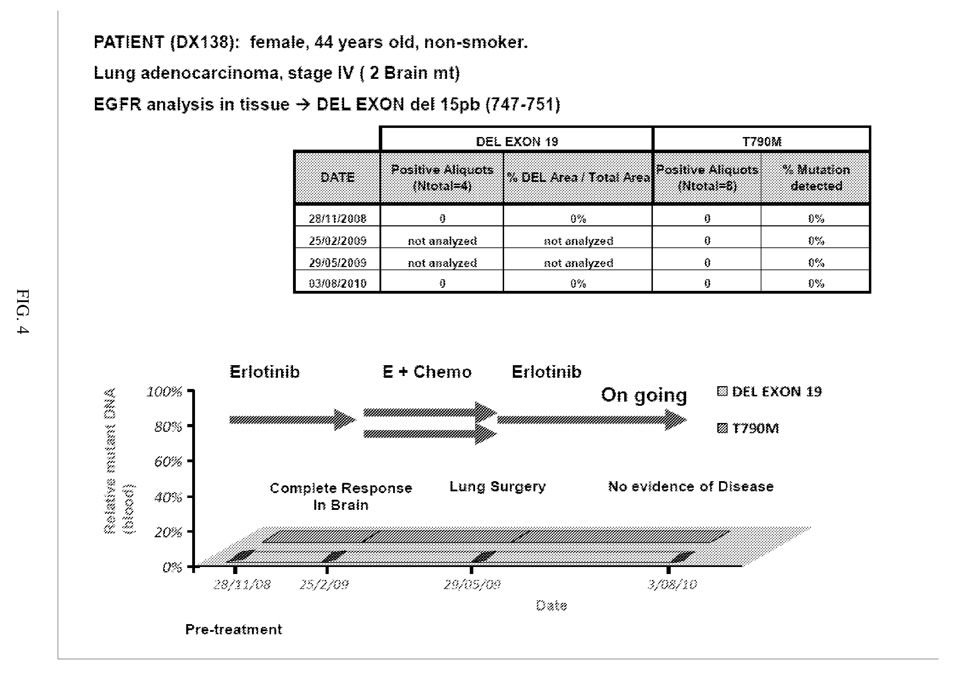

Patient DX 138 (Fig. 4); this is an example of a sensitive patient; no detection of the EGFR mutation in serum at baseline and during therapy (yet) the patient continues to be monitored while on Erlotininb therapy and still responding. This is example of the value of the test as follow-up (for disease recurrence) in disease free patients that are on therapy with EGFR TK inhibitors.

## Claims

1. A method for predicting the clinical response of a patient suffering lung cancer to an EGFR inhibitor-based therapy comprising
(i) determining at a first time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid sequence of the non mutated EGFR gene and
(ii) determining at a second time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid of the non mutated EGFR gene,
wherein said second time point is later than the first time point and
wherein an increase in the ratio determined at the second time point with respect to the ratio determined at the first time point is indicative of a negative clinical response of said patient to said EGFR inhibitor-based therapy or
wherein a decrease in the ratio determined at the second time point with respect to the ratio at determined at the first time point is indicative of a positive clinical response of said patient to said EGFR inhibitor-based therapy.

2. A method as defined in claim 1 further comprising determining at said first and second time points the ratio between the number of copies of the nucleic acid sequence of the EGFR gene which contains at least one mutation conferring resistance of EGFR towards an inhibitor of EGFR tyrosine kinase activity and the number of copies of the nucleic acid of the non mutated EGFR gene in the biofluid of the patient
wherein an increase in the ratio determined at the second time point with respect to the ratio at determined at the first time point is indicative of a negative response or wherein a decrease in the ratio determined at the second time point with respect to the ratio at determined at the first time point is indicative of a positive response.

3. A method as defined in claim 2 wherein the resistance mutation of the EGFR gene towards an inhibitor of tyrosine kinase activity is the T790M mutation in exon 20.

4. A method according to any of claims 1 to 3 wherein a tissue sample from the tumor of the patient at the first time point is positive for a sensitivity mutation of the EGFR gene towards an inhibitor of tyrosine kinase activity.

5. A method according to any of claims 1 to 4, wherein the EGFR inhibitor-based therapy started prior to the first time point or between the first and the second time point.

6. A composition comprising an EGFR inhibitor for use in the treatment of lung cancer in a patient suffering lung cancer wherein said patient is selected by a method comprising
(i) determining at a first time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid sequence of the non mutated EGFR gene and
(ii) determining at a second time point in a bio-fluid of said patient the ratio between the number of copies of the nucleic acid sequence of the EGFR gene which contains at least one mutation conferring sensitivity of EGFR towards an inhibitor of EGFR tyrosine kinase activity and the number of copies of the nucleic acid of the non mutated EGFR gene,
wherein said second time point is later than the first time point and
wherein the ratio determined at the second time point is increased with respect to the ratio at determined at the first time point.

7. A composition for use as defined in claim 6 wherein the method for selecting the patient further comprises determining at said first and second time points the ratio between the number of copies of the nucleic acid sequence of the EGFR gene which contains at least one mutation conferring resistance of EGFR towards an inhibitor of EGFR tyrosine kinase activity and the number of copies of the nucleic acid of the non mutated EGFR gene in the biofluid of the patient and wherein the patient is further selected wherein an increase in the ratio determined at the second time point with respect to the ratio determined at the first time is determined.

8. A composition for use according to claim 7 wherein the resistance mutation of the EGFR gene towards an inhibitor of tyrosine kinase activity is the T790M mutation in exon 20.

9. A composition for use according to any of claims 6 to 8 wherein the method further comprises selecting those patients wherein a tissue sample from the tumor at the first time point is positive for a sensitivity mutation of the EGFR gene towards an inhibitor of tyrosine kinase activity.

10. A method as defined in any of claims 1 to 5 or a composition for use according to any of claims 6 to 9 wherein lung cancer is advanced lung cancer or Non Small Cell Lung Cancer.

11. A method as defined in any of claims 1 to 5 or 10 or a composition for use according to any of claims 6 to 10 wherein the biofluid is serum.

12. A method as defined in any of claims 1 to 5, 10 or 11 or a composition for use according to any of claims 6 to 11 wherein the EGFR inhibitor-based therapy comprises a EGFR tyrosine-kinase inhibitor.

13. A method or a composition for use according to claim 12 wherein the EGFR tyrosine-kinase inhibitor is erlotinib or gefitinib.

14. A method as defined in any of claims 1 to 5 or 10 to 13 or a composition for use according to any of claims 6 to 13 wherein the sensitivity mutation of the EGFR gene towards an inhibitor of tyrosine kinase activity, is selected from L858R mutation and an (E)LREA deletion in exon 19.

15. A method as defined in any of claims 1 to 5 or 10 to 14 or a composition for use according to any of claims 6 to 14 wherein the patient has no relapse symptoms at the first and/or second time points.


SEQUENCE LISTING

<110>  PANGAEA BIOTECH S.L.

<120>  MOLECULAR BIOMARKERS FOR PREDICTING RESPONSE TO TYROSINE   KINASE
       INHIBITORS IN LUNG CANCER

<130>  P6460EP00

<160>  15

<170>  PatentIn version 3.5

<210>  1
<211>  20
<212>  DNA
<213>  Artificial

<220>
<223>  Primer

<400>  1
gtgcatcgct ggtaacatcc                                                   20


<210>  2
<211>  20
<212>  DNA
<213>  Artificial

<220>
<223>  Primer

<400>  2
tgtggagatg agcagggtct                                                   20


<210>  3
<211>  17
<212>  DNA
<213>  Artificial

<220>
<223>  Probe

<400>  3
agatgttgct tctctta                                                      17


<210>  4
<211>  22
<212>  DNA
<213>  Artificial

<220>
<223>  Primer

<400>  4

actctggatc ccagaaggtg ag                                                22

<210>  5
<211>  22
<212>  DNA
<213>  Artificial

<220>
<223>  Primer

<400>  5
ccacacagca aagcagaaac tc                                                22

<210>  6
<211>  20
<212>  DNA
<213>  Artificial

<220>
<223>  Primer

<400>  6
aacaccgcag catgtcaaga                                                   20

<210>  7
<211>  18
<212>  DNA
<213>  Artificial

<220>
<223>  Primer

<400>  7
ttctcttccg cacccagc                                                     18

<210>  8
<211>  21
<212>  DNA
<213>  Artificial

<220>
<223>  Probe

<400>  8
cagattttgg gcgggccaaa c                                                 21

<210>  9
<211>  24
<212>  DNA
<213>  Artificial

<220>
<223>  Probe

<400>  9
tcacagattt tgggctggcc aaac                                              24


<210>  10
<211>  15
<212>  DNA
<213>  Artificial

<220>
<223>  Probe

<400>  10
agtttggcca gccca                                                        15


<210>  11
<211>  16
<212>  DNA
<213>  Artificial

<220>
<223>  Primer

<400>  11
aggcagccga agggca                                                       16


<210>  12
<211>  18
<212>  DNA
<213>  Artificial

<220>
<223>  Primer

<400>  12
cctcacctcc accgtgca                                                     18


<210>  13
<211>  18
<212>  DNA
<213>  Artificial

<220>
<223>  Probe

<400>  13
ctcatcacgc agctcatg                                                     18


<210>  14
<211>  18
<212>  DNA
<213>  Artificial

<220>
<223>  Probe

<400>  14
ctcatcatgc agctcatg                                                    18

<210>  15
<211>  14
<212>  DNA
<213>  Artificial

<220>
<223>  Probe

<400>  15
tcatcacgca gctc                                                        14